(19) Europäisches Patentamt
European Patent Office
Office européen des brevets

(11) EP 1 651 777 B1

(12) **EUROPEAN PATENT SPECIFICATION**

(45) Date of publication and mention of the grant of the patent:
**16.07.2008 Bulletin 2008/29**

(21) Application number: **04778518.3**

(22) Date of filing: **16.07.2004**

(51) Int Cl.:
***C12Q 1/68*** *(2006.01)*

(86) International application number:
**PCT/US2004/023050**

(87) International publication number:
**WO 2005/017204 (24.02.2005 Gazette 2005/08)**

(54) **USE SINGLE NUCLEOTIDE POLYMORPHSM IN THE CODING REGION OF THE PORCINE LEPTIN RECEPTOR GENE TO ENHANCE PORK PRODUCTION**

VERWENDUNG EINES EINZELNUKLEOTID-POLYMORPHISMUS IM CODIERENDEN BEREICH DES LEPTINREZEPTOR-GENS AUS SCHWEIN ZUR VERBESSERUNG DER SCHWEINEFLEISCHPRODUKTION

UTILISATION DE POLYMORPHISME NUCLEOTIDIQUE UNIQUE DANS LA REGION DE CODAGE DU GENE DE RECEPTEUR DE LEPTINE PORCIN PERMETTANT D'AUGMENTER LA PRODUCTION EN ELEVAGE PORCIN

(84) Designated Contracting States:
**AT BE BG CH CY CZ DE DK EE ES FI FR GB GR HU IE IT LI LU MC NL PL PT RO SE SI SK TR**

(30) Priority: **07.08.2003 US 493158 P**
**16.03.2004 US 553582 P**

(43) Date of publication of application:
**03.05.2006 Bulletin 2006/18**

(73) Proprietor: **Newsham Choice Genetics, LLC**
**St Louis, MO 63167 (US)**

(72) Inventors:
- **KOJIMA, Cheryl J.**
  **Knoxville, TN 37920 (US)**
- **DU, Fengxing**
  **St. Charles, MO 63304 (US)**
- **GROSZ, Michael, D.**
  **Ellisville, MO 63021 (US)**
- **BYATT, John, C.**
  **Ballwin, MO 63011 (US)**

(74) Representative: **Holmberg, Martin Tor et al**
**Bergenstrahle & Lindvall AB**
**P.O. Box 17704**
**118 93 Stockholm (SE)**

(56) References cited:
**US-B1- 6 458 531**

- **STRATIL A ET AL: "HpaII and RsaI PCR-RFLPs within an intron of the porcine leptin receptor gene (LEPR) and its linkage mapping" ANIMAL GENETICS, vol. 29, no. 5, October 1998 (1998-10), pages 405-406, XP002320727 ISSN: 0268-9146**
- **VINCENT A L ET AL: "Rapid communication: A restriction fragment length polymorphism in the porcine leptin receptor (LEPR) gene" JOURNAL OF ANIMAL SCIENCE, vol. 75, no. 8, 1997, page 2287, XP002320728 ISSN: 0021-8812**
- **RUIZ-CORTES Z TATIANA ET AL: "Porcine leptin receptor: Molecular structure and expression in the ovary" MOLECULAR REPRODUCTION AND DEVELOPMENT, vol. 56, no. 4, August 2000 (2000-08), pages 465-474, XP002320729 ISSN: 1040-452X & DATABASE EMBL [Online] 29 September 1998 (1998-09-29), "Sus scrofa transmembrane leptin receptor (LEPR) mRNA, LEPR-Rb allele, complete cds." retrieved from EBI accession no. EM_PRO:AF092422 Database accession no. AF092422**
- **DATABASE EMBL [Online] 2 August 2000 (2000-08-02), "Sus scrofa transmembrane leptin receptor (OBR) mRNA, OBR-db allele, partial cds." XP002320733 retrieved from EBI accession no. EM_PRO:AF167719 Database accession no. AF167719**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KORWIN-KOSSAKOWSKA AGNIESZKA ET AL: "The effect of the polymorphism of leptin (LEP), leptin receptor (LEPR) and osteopontin (OPN) genes on selected reproduction traits of synthetic Line 990 sows" ANIMAL SCIENCE PAPERS AND REPORTS, vol. 20, no. 3, 2002, pages 159-168, XP008044159 ISSN: 0860-4037
- SWITONSKI MAREK ET AL: "Searching for genes controlling fatness traits in pigs: A review." ANIMAL SCIENCE PAPERS AND REPORTS, vol. 21, no. 2, 2003, pages 73-86, XP008044189 ISSN: 0860-4037
- DATABASE BIOSIS [Online] BIOSCIENCES INFORMATION SERVICE, PHILADELPHIA, PA, US; 2000, KORWIN-KOSSAKOWSKA AGNIESZKA: "[Genes for reproductive traits in pigs: A review]" XP002320732 Database accession no. PREV200100323124 & PRACE I MATERIALY ZOOTECHNICZNE, no. 57, 2000, pages 25-37, ISSN: 0137-1649

SEQUENCE LISTING

<110> Dyer, Cheryl J.
Du, Fengxing
Grosz, Michael D.
Byatt, John C.
<120> USE OF A SINGLE NUCLEOTIDE POLYMORPHISM IN THE CODING REGION OF THE LEPTIN RECEPTOR GENE TO ENHANCE PORK PRODUCTION
<130> 11916.0058.00PC01
<150> US. 60/553,582
<151> 2004-03-16
<150> U.S. 60/493,158
<151> 2003-08-07
<160> 44
<170> PatentIn version 3.2
<210> 1
<211> 21
<212> DNA
<213> Artificial Sequence
<220>
<223> Synthetic nucleotide
<400> 1
atgatgaggc agttgttgca a 21
<210> 2
<211> 20
<212> DNA
<213> Artificial Sequence

<220>
<223> Synthetic nucleotide
<400> 2
ccttccctgc aatgttgtct 20
<210> 3
<211> 773
<212> DNA
<213> Sus scrofa
<400> 3

```
gtgggttaag gacctgatgt tgtcactact atggctcgag tcactgctgg ggcatgagtt     60
tgatccctgg tcctggaaat tcacatgctg tgcatgtggc catatatata tgtatgtatg    120
tgtatatata tacactcaca tacatgtata tatatatatg tgagtgtata tatatattta    180
tgatgtcaaa ttaatgggga aaataaaatg tgaatttcta aaaaggggtg ctaaagagtg    240
gcattatctc taagggtata tgctccctct taagtataac actttggaca atggaagagc    300
tttgtattag gcactgtttg agcacttgga aagttaaata attattgttg aagactgcat    360
gttttaatct tagatacttc ctatttatgt cttagtcaaa atgattaatt gcttttctat    420
gtgtctttta aatgtcctaa cagaatttat ttatgtgata actgcatttg acttggcata    480
tccaattact ccttggaaat ttaagttgtc ttgcatgcca ccaaatacaa catatgactt    540
cctcttgcct gctggaatct caaagaacac ttcaactttg aatggacatg atgaggcagt    600
tgttgaaacg gaacttaatt caagtggtac ctacttatca aacttatctt ctaaaacaac    660
tttccactgt tgcttttgga gtgaggaaga taaaaactgc tctgtacatg cagacaacat    720
tgcagggaag gcatttgttt cagcagtaaa ttccttagtt tttcaacaaa cag           773
```

<210> 4
<211> 20
<212> DNA
<213> Artificial Sequence
<220>
<223> Synthetic nucleotide
<400> 4
gcactgtttg agcacttgga 20
<210> 5
<211> 20
<212> DNA
<213> Artificial Sequence
<220>
<223> Synthetic nucleotide
<400> 5
ccttccctgc aatgttgtct 20
<210> 6
<211> 25
<212> DNA

<213> Artificial Sequence
<220>
<223> Synthetic nucleotide
<400> 6
ttcaactttg aatggacatg atgag 25
<210> 7
<211> 30
<212> DNA
<213> Artificial Sequence
<220>
<223> Synthetic nucleotide
<400> 7
gtggaaagtt gttttagaag ataagtttga 30
<210> 8
<211> 16
<212> DNA
<213> Artificial Sequence
<220>
<223> Synthetic nucleotide
<400> 8
tgttgaaacg gaactt 16
<210> 9
<211> 17
<212> DNA
<213> Artificial Sequence
<220>
<223> Synthetic nucleotide
<400> 9
tgttgaaatg gaactta 17
<210> 10
<211> 421
<212> DNA
<213> Sus scrofa
<220>
<221> CDS
<222> (133)..(420)
<220>
<221> misc_feature
<222> (299)..(299)
<223> N = T or C
<220>
<221> misc_feature
<222> (310)..(310)
<223> N = T or A
<220>
<221> misc_feature
<222> (311)..(311)
<223> N = T or C
<400> 10

```
gcactgtttg agcacttgga aagttaaata attattgttg gagactgcat gttttaatct      60

tagatacttc ctatttatgt cttagtcaaa atgattaatt gcttttctat gtgtctttta     120

aatgtcctaa ca gaa ttt att tat gtg ata act gca ttt gac ttg gca tat     171
              Glu Phe Ile Tyr Val Ile Thr Ala Phe Asp Leu Ala Tyr
              1               5                   10

cca att act cct tgg aaa ttt aag ttg tct tgc atg cca cca aat aca       219
Pro Ile Thr Pro Trp Lys Phe Lys Leu Ser Cys Met Pro Pro Asn Thr
    15                  20                  25

aca tat gac ttc ctc ttg cct gct gga atc tca aag aac act tca act       267
Thr Tyr Asp Phe Leu Leu Pro Ala Gly Ile Ser Lys Asn Thr Ser Thr
30                  35                  40                  45

ttg aat gga cat gat gag gca gtt gtt gaa ang gaa ctt aat nna agt       315
Leu Asn Gly His Asp Glu Ala Val Val Glu Xaa Glu Leu Asn Xaa Ser
                50                  55                  60

ggt acc tac tta tca aac tta tct tct aaa aca act ttc cac tgt tgc       363
Gly Thr Tyr Leu Ser Asn Leu Ser Ser Lys Thr Thr Phe His Cys Cys
            65                  70                  75

ttt tgg agt gag gaa gat aaa aac tgc tct gta cat gca gac aac att       411
Phe Trp Ser Glu Glu Asp Lys Asn Cys Ser Val His Ala Asp Asn Ile
        80                  85                  90

gca ggg aag g                                                         421
Ala Gly Lys
    95
```

<210> 11
<211> 96
<212> PRT
<213> Sus scrofa
<220>
<221> misc_feature
<222> (56)..(56)
<223> The 'Xaa' at location 56 stands for Thr or Met.
<220>
<221> misc_feature
<222> (60)..(60)
<223> The 'Xaa' at location 60 stands for Ile or Ser.
<400> 11

```
Glu Phe Ile Tyr Val Ile Thr Ala Phe Asp Leu Ala Tyr Pro Ile Thr
```

```
1               5                   10                  15

Pro Trp Lys Phe Lys Leu Ser Cys Met Pro Pro Asn Thr Thr Tyr Asp
            20                  25                  30

Phe Leu Leu Pro Ala Gly Ile Ser Lys Asn Thr Ser Thr Leu Asn Gly
        35                  40                  45

His Asp Glu Ala Val Val Glu Xaa Glu Leu Asn Xaa Ser Gly Thr Tyr
    50                  55                  60

Leu Ser Asn Leu Ser Ser Lys Thr Thr Phe His Cys Cys Phe Trp Ser
65                  70                  75                  80

Glu Glu Asp Lys Asn Cys Ser Val His Ala Asp Asn Ile Ala Gly Lys
                85                  90                  95
```

<210> 12
<211> 4050
<212> DNA
<213> Sus scrofa
<400> 12

```
cttctctgaa gtaagatgac gtgtccaaag ttctctgtgg ctttgttaca ttgggaattt     60
atttatgtga taactgcatt tgacttggca tatccaatta ctccttggaa atttaagttg    120
tcttgcatgc caccaaatac aacatatgac ttcctcttgc ctgctggaat ctcaaagaac    180
acttcaactt tgaatggaca tgatgaggca gttgttgaaa cggaacttaa tataagtggt    240
acctacttat caaacttatc ttctaaaaca actttccact gttgcttttg gagtgaggaa    300
gataaaaact gctctgtaca tgcagacaac attgcaggga aggcatttgt ttcagcagta    360
aattccttag tttttcaaca aacaggtgca aactggaaca tacagtgctg gatgaaagag    420
gacttgaaat tattcatctg ttatatggag tcattattta agaatccttt caagaattat    480
gaccttaaag ttcatctttt atatgttctg ctcgaagtgt tagaaggatc acctctgctc    540
ccccagaaag gtagttttca gagcgttcaa tgcaactgca gtgctcgtga atgttgtgaa    600
tgccatgtgc ctgtgtcggc agccaaactc aactacaccc ttcttatgta tttgaaaatc    660
acatctggtg gagcagtttt tcactcacct ctcatgtcag ttcagcccat aaacgttgtg    720
aagcctgatc caccattagg tttgcatatg gaaatcacag acactggtaa tttaaagatt    780
tcttggtcca gcccaacact ggtaccattt caacttcaat atcaagtaaa atattcagag    840
```

```
aattctacaa caaatatgag agaagctgat gagatcgtct cagatacatc tctgcttgta     900
gacagtgtgc ttcccgggtc ttcatatgag gttcaggtga ggggcaagag actggatggc     960
ccaggaatct ggagtgactg gagcaccccc tttacttta ccacacaaga tgttatatac     1020
tttccaccta aaattctgac aagtgttggg tctaacattt cttttcactg catctataaa    1080
aatgagaaca agatcgtttc ctcaaaaaag attgtttggt ggatgaattt agctgagaag    1140
attcctcaaa gtcagtatga tgttgtgggt gaccatgtta gcaaagtcac ttttcccaat    1200
atgaatgcaa ccaaacctcg aggaaagttc acctatgatg cagtgtactg ctgcaatgag    1260
cacgagtgcc accatcgcta tgctgagtta tatgtgattg atgtcaatat caatatatca    1320
tgtgaaactg atgggtactt aactaaaatg acttgcagat ggtcaaccaa tgcaatccaa    1380
tcacttgtgg gaagcacttt gcagttgagg tatcatagga gtagcctcta ctgttctgac    1440
gttccatctg tgcatcccat atctgaaccc aaagattgcc agttgcagag agatggtttt    1500
tatgaatgca tatttcagcc aatatttctg ctatctggct atacaatgtg gattagaata    1560
aatcacccgt tgggttcact tgattctcca ccaacatgtg tcattcctga ttccgtggtg    1620
aaaccgctgc ctccatccag tgtgaaagca gaaattactg caaaaattgg attactgaaa    1680
atatcttggg agaagccagt cttcccagag aataatcttc agttccagat tcgctatggt    1740
ttaagtggaa aagaagtaca gtggaagatc tatgaggtat atgacacaaa gttaaaatcc    1800
accagtctcc cggtgccaga cctgtgtgca gtctatgctg tccaggtgcg ctgtaagagg    1860
ctagatggac tgggctattg gagtaattgg agtactccag cctacacagt tgtcacggat    1920
gtaaaagttc ctatcagagg acctgaattt tggagaataa ttaatgaaga tgccactaaa    1980
aaagagagga atatcactct gctctggaag cctctgatga aaaatgactc attgtgcagc    2040
gtgagaagtt atgtggtgaa acatcatact tcccgccatg gaacatggtc agaagatgtg    2100
ggaaaccaca ctaaactcac tttcctttgg acagagcaag cacattctgt tacagttctg    2160
gccgtcaatt caattggtgc ttcttccgca aattttaatt taacattctc atggcccatg    2220
agcaaagtaa atatcgtgca gtcgctcagt gcttatcctt taaacagcag ttgtgtgggt    2280
ctttcctggc tgctctcacc cagtgattac aatctgatgt attttattct tgagtggaaa    2340
attcttaatg aagaccatga aattaaatgg ctcagaatcc cttcctctgt taaaaagtat    2400
tatatccacg atcattttat tcctattgag aaatatcaat tcagtcttta ccccatattc    2460
atggaaggag tggggaaacc gaagataatt aacagtttca cccaagatgg tgaaaaacac    2520
cggaatgatg caggtctata tgtaattgtg ccaataatta tttcctcttc aatcttattg    2580
```

```
cttggaacat tgttaatgtc acaccaaaga atgaaaaagc tattttggga agatgttcca   2640
aaccccaaga actgttcctg ggcacaagga cttaattttc agaagccgga aacatttgag   2700
catcttttta tcaagcacac agaatcagtg acatttggcc ctcttctttt ggagcctgaa   2760
accatttcag aagatatcag tgttgataca tcatggaaaa ataaggatga gatggtgcca   2820
ccaactacag tctctctact cttgacaact ccggaccttg aaaagagttc aatttgtatt   2880
agtgaccaac gcagcagtgc ccacttctct gaggctgaga gcatggagat aactcgtgag   2940
gatgaaaata gaagacagcc ctctattaaa tatgccaccc tgctcagcag ccctaaatca   3000
ggtgaaactg agcaagagca agaacttgta agtagcttgg tcagcagatg cttctctagc   3060
agcaattccc taccgaaaga gtctttctcg aatagctcat gggagataga aacccaggcc   3120
tttttttattt tatcagatca gcatcccaat atgacttcac cacacctttc cttctcagaa   3180
ggattggatg aacttatgaa gtttgaggga aatttcccca aagaacataa tgacgaaagg   3240
tctgtctatt atttaggagt cacctcaatc aaaaagagag agagtgatgt gtttttgact   3300
gatgagtcaa gagtgcggtg cccattccca gcccactgtt tattcgctga catcaaaatc   3360
ctccaggaga gctgttcaca ccttgtagaa aataatttca atttaggaac ttctggtcag   3420
aagacttttg tatcttacat gcctcaattt caaacttgtt caactcagac tcagaagata   3480
atggaaaaca agatgtatga cctaaccgtc taagttcatt ccagaaacat ctcagattta   3540
tgatgggatg agtcatatta agggtaatat gttctacatg gtgttccata gcagagagaa   3600
aaaaattgag tcaaatttga aaatgacttc aaaagttaaa gagatctgtt tgtccacact   3660
cagtaataca gaaaaaaaaa tgtgagaaag ccttcaagag cctagtaatg tagacctact   3720
cttctaatga ttctcttaac cggctacagt gggaagttct cgaatgcctt gtgtctagct   3780
agaaacaagc ccaacaatac tagcgttttg agcattaatc tcatgtagaa agagctaatc   3840
catctgaatt acacatacat ctgaaagaag acttcagact aacacttgtg aaatgtaatg   3900
tcttcaagag tgtgattgtt ttatcttgag gtgtctttgt tttacactaa tttacacata   3960
cacatatgca cacttgtatc taataggcat cctgtacatt gttaaatata tgatgtactt   4020
gtttttgtgc taaaaaaaaa aaaaaaaaaa                                     4050
```

<210> 13
<211> 1025
<212> DNA
<213> Sus scrofa
<220>
<221> misc_feature
<222> (1)..(1025)
<223> N = unknown
<400> 13

```
tgcagtgtga cttgaagcat ttggcacatt gttcaagttc acacaagccc tatgggcaca      60
acttttaaac ctaatctttt tatgatgcca accaaagtag ctttgaatct ggcatcaatt     120
tgcagaggaa gtttattttc ccttagcttt tgcgtcgtta aaatgattac tcctgaggaa     180
atatgaccct acatttggta tttggaaaca gggagtcagt tttattggaa agggatgaga     240
gggggtagaa gaatgtcatg cttagggttg taaaacctta ttcttggtcc aggatcaccc     300
actggttggg gagtttcatc caagatgttt cactacttga gactaggctt aaaaataaaa     360
ggctgtttct attcctctgg tcaatatgta gctcatctct aaacaggaac atagggctcc     420
aatangannn ccccagtctt gtagttaagt gtaccttaac tttttgcttc ttotttcttc     480
ttannagctt taacttanna aatattgtca tcttgttaac cctgacnnat gatttatctt     540
catcaatctg tttagacttg aagtcanngc tcaaattann ttctgnnntt tcatnnngnn     600
cnnnnntngn nnnnnnnnnn nnnagcttgt gtgccaattt nnnnnnnnnn natgaantac     660
tcnnannnnn nnnnnnnnnn nnngnnnaaa nnnnnnnnnn nnnncnncnn nnnnnnnnnn     720
nnnnnnnnnn nnnnnnnnnn nnnnnnnnnn nnnnnnnnnn nnnnnnnnnn nnnnnnnnnn     780
nnnnnnnnnn nnnnnnnnnn nnnnnnnnnn nnnnnnnnnn nnnnnnnnnn nnnnnnnnnn     840
nnnnnnnnnn nnnncagnnt natgaannnn nnctanannn nnncnacttg gacctggggc     900
actattgtgg tctcaggagt tctgttccca ggattcagga attcactaga gtgtacacag     960
agcatgacaa aaccttgggc tgactggacc atttatcagt ttctctttcc tgttgttcta    1020
ggtcc                                                                1025
```

<210> 14
<211> 446
<212> DNA
<213> Sus scrofa
<220>
<221> misc_feature
<222> (1)..(446)
<223> N = unknown
<400> 14

```
caggaattcg gcaccagaca taatgtaatg ttttgtaagt tattaattta tatatctaac      60
```

attgcctgcc aatggtggtg ttaaatttgt gtagaagact ctgcctaaga gttgcgactt 120
ttcttgtaat gttttgtatc gtgtattata taacctgaac atcgcttaag agagacatac 180
acccccgcc ccttgccagc gaggacagca gtgggtctgc cctacgcctt gtccgagttg 240
ctaatattcc tcaacccctt caccaaccgg tttgggaaac aggattctca cgttagatac 300
gaaatggtct cgattgagct tttacttttg tatagttcaa caggggtaga gagccatggg 360
acatggtttt acccctgttc tacccaaatc catatacatg cgnnggnnnt taactggnnn 420
ctactataat tnnnnntttt cnnnnt 446

<210> 15
<211> 770
<212> DNA
<213> Sus scrofa
<220>
<221> misc_feature
<222> (1)..(770)
<223> N = unknown
<400> 15

caaggaagag aagctaaggc aagatttcaa aaacagaaat ccaagaattc cagcaaacca 60
gggttagatt catagtacaa ggtctatgat atatttgagc tacaagaagg ttttctaggc 120
aacagaatat caaaagaggg gtaaagccta catatcttca gtctaaaaaa tgaagttata 180
aaactcttag tgtcttaagc tatgttttca acagaccctc tgatatttgg aaaagcagag 240
gaaaatttgg aagcccactg ttgcaatcaa caggagctac taaaatttta gtctattttt 300
ttcaactcta tcagttcttt tcttatactc aaatgattat cctggctatt aaataatctc 360
tttcctccct ccacacaccc gctgccagtg gactctcctt ttatatattt tactttttga 420
attcaagtct tctatatctt agtacaatgg ccaaaaaaac taagctttct aaggcaccca 480
agagttagaa cttttcattt cctacttcat atgcaagaaa ttttctctcc ctttgtctac 540
ttcataagta atgattagca atgggtaaat atcaaaagag ctaacggtag actatatttt 600
aggcatggaa taatttccct taatagacat tatccagtag ccccctctta ttggcagnnn 660
atatgtnnnn ngnnnctcag tngatgccnn nnnctnnnnn tngtactgaa cgctacatat 720
gctattcttt nntatacant catanntatg nnnanncnnn actnacnnan 770

<210> 16
<211> 362
<212> DNA
<213> Sus scrofa
<400> 16

gggaccgtca gtgtgaccaa atcagggcgc cagtgccagc cgtggaattc ccaatatccc 60

cacacacaca ccttcaccgc cctccgtttc ccagaactga atggagggca ctcctattgc 120

cgcaacccag ggaatcagaa ggaagctccc tggtgcttca ccttggatga gaactttaag 180

tccgacctgt gtgacatccc agcatgtgat tcaaaggatt ccaaagagaa gaataaaatg 240

gaaatcctgt acatactggt gcccagtgtt gccatccccc tggccattgc cttactcttc 300

ttcttcatct gtgtctgtcg caataaccag aagtcgtcct caccggctgt ccagaggcaa 360

cc 362

<210> 17
<211> 625
<212> DNA
<213> Sus scrofa
<400> 17

gtacacagat gtaaaaacac ttagtgttca cacgtttgat ttaaatattg acaaattttt 60

tcattagtac attaaacctt tcgctttatt catcttaaat gtcttccagg agggtgactc 120

cccccattag cgtgactcaa tacaaacttt gcaagtgggg ggaccacgga acccggaagt 180

ctactgctgt gcccgttcta tggcgaggca gctgtaactg gttacgaacc cgtgttggaa 240

atagtatttg gaactttctt ggcagatttc ttacatcgtt attcaatatg agctgcgaat 300

catatgctcg tagttaggaa aatgtcagga aaccctgagt gtgcctgctt tgtttgacaa 360

agctattttc gagtcatgtt ggaaggcaag ggcatccagc gcctggcatg gaggagaaga 420

gggtagccct gcccccacc ttcccagcct ttttctgaga tgttggtaat tcggtcctag 480

atgacaagcg ctcaactctg aacaagagac ggccatctca caccgtctca attagtccag 540

gatgtgtgtc agggctgcga gaggtcggag aggaaatgcg gggaacttgt tcacttcttg 600

ctcagtttgg atcaactgag ctgca 625

<210> 18
<211> 22
<212> DNA
<213> Artificial Sequence
<220>
<223> Synthetic nucleotide
<400> 18
ggcagctgta actggttacg aa 22
<210> 19
<211> 26
<212> DNA
<213> Artificial Sequence
<220>
<223> Synthetic nucleotide
<400> 19
tcgcagctca tattgaataa cgatgt 26

<210> 20
<211> 19
<212> DNA
<213> Artificial Sequence
<220>
<223> Synthetic nucleotide
<400> 20
aagttccaaa tactctttc 19
<210> 21
<211> 19
<212> DNA
<213> Artificial Sequence
<220>
<223> Synthetic nucleotide
<400> 21
aagttccaaa tactatttc 19
<210> 22
<211> 26
<212> DNA
<213> Artificial Sequence
<220>
<223> Synthetic nucleotide
<400> 22
cagaccctct gatatttgga aaagca 26
<210> 23
<211> 33
<212> DNA
<213> Artificial Sequence
<220>
<223> Synthetic nucleotide
<400> 23
gccaggataa tcatttgagt ataagaaaag aac 33
<210> 24
<211> 17
<212> DNA
<213> Artificial Sequence
<220>
<223> Synthetic nucleotide
<400> 24
acaggagcta ctaaaat 17
<210> 25
<211> 16
<212> DNA
<213> Artificial Sequence
<220>
<223> Synthetic nucleotide
<400> 25
caggagctat taaaat 16
<210> 26
<211> 25
<212> DNA
<213> Artificial Sequence
<220>
<223> Synthetic nucleotide
<400> 26
acattctaag acaaccgaaa tggca 25
<210> 27
<211> 34

<212> DNA
<213> Artificial Sequence
<220>
<223> Synthetic nucleotide
<400> 27
ctagggatct atttttcact tttgtaagtt catt 34
<210> 28
<211> 25
<212> DNA
<213> Artificial Sequence
<220>
<223> Synthetic nucleotide
<400> 28
ataattttca taaagaccca ctaat 25
<210> 29
<211> 17
<212> DNA
<213> Artificial Sequence
<220>
<223> Synthetic nucleotide
<400> 29
cataaaggcc cactaat 17
<210> 30
<211> 25
<212> DNA
<213> Artificial Sequence
<220>
<223> Synthetic nucleotide
<400> 30
taaatgtctt ccaggagggt gactc 25
<210> 31
<211> 25
<212> DNA
<213> Artificial Sequence
<220>
<223> Synthetic nucleotide
<400> 31
cacacatcct ggactaattg agacg 25
<210> 32
<211> 22
<212> DNA
<213> Artificial Sequence
<220>
<223> Synthetic nucleotide
<400> 32
caagaattcc agcaaaccag gg 22
<210> 33
<211> 25
<212> DNA
<213> Artificial Sequence
<220>
<223> Synthetic nucleotide
<400> 33
ctcttgggtg ccttagaaag cttag 25
<210> 34
<211> 25
<212> DNA
<213> Artificial Sequence

<220>
<223> Synthetic nucleotide
<400> 34
gggagtttca tccaagatgt ttcac 25
<210> 35
<211> 25
<212> DNA
<213> Artificial Sequence
<220>
<223> Synthetic nucleotide
<400> 35
aaactgataa atggtccagt cagcc 25
<210> 36
<211> 20
<212> DNA
<213> Artificial Sequence
<220>
<223> Synthetic nucleotide
<400> 36
ttcccaatat ccccacacac 20
<210> 37
<211> 20
<212> DNA
<213> Artificial Sequence
<220>
<223> Synthetic nucleotide
<400> 37
gctgggatgt cacacaggtc 20
<210> 38
<211> 20
<212> DNA
<213> Artificial Sequence
<220>
<223> Synthetic nucleotide
<400> 38
tgggaaacag gattctcacg 20
<210> 39
<211> 20
<212> DNA
<213> Artificial Sequence
<220>
<223> Synthetic nucleotide
<400> 39
tggatttggg tagaacaggg 20
<210> 40
<211> 20
<212> DNA
<213> Artificial Sequence
<220>
<223> Synthetic nucleotide
<400> 40
cagcagccct aaatcaggtg 20
<210> 41
<211> 20
<212> DNA
<213> Artificial Sequence
<220>
<223> Synthetic nucleotide

<400> 41
aggcctgggt ttctatctcc 20
<210> 42
<211> 406
<212> DNA
<213> Sus scrofa
<220>
<221> misc_feature
<222> (103)..(103)
<223> N = T or G
<400> 42

```
tcatacaact ttgcagtggg gggaccacgg aacccggaag tctactgttg tgcccgttct     60
atggtgaggc agctgtaact ggttacgaac ccgtgttgga aanagtattt ggaactttct    120
tggcagattt cttacatcgt tattcaatat gagctgcgaa tcatatgctc gtagttagga    180
aaatgtcagg aaaccccgag tgtgcctgct ttgtttgaca aagctatttt cgagtcatgt    240
tggaaggcaa gggcatccag cgcctggcat ggaggagaag agggtagccc tgccccccac    300
cttcccagcc tttttctgag atgttggtaa ttcggtccta gatgacaagc gctcaactct    360
gaacaaggga cggccgtctc acaccgtctc aattagtcca ggatgt                   406
```

<210> 43
<211> 395
<212> DNA
<213> Sus scrofa
<220>
<221> misc_feature
<222> (192)..(192)
<223> N = T or C
<400> 43

```
gatatatttg agctacagaa ggttttctag gcaacagaat atcaaaagag gggtaaagcc     60
tacatatctt cagtctaaaa aatgaagtta taaaactctt agtgtcttaa gctatgtttt    120
caacagaccc tctgatattt ggaaaagcag aggaaaattt ggaagcccac tgttgcaatc    180
aacaggagct antaaaattt tagtctattt tttcaactct atcagttctt ttcttatact    240
caaatgatta tcctggctat taaataatct ctttcctccc tccacacacc cgctgccagt    300
ggactctcct tttatatatt ttactttttg aattcaagtc ttctatatct tagtacaatg    360
gccaaaaaaa ctaagctttc taaggcaccc aagag                               395
```

<210> 44
<211> 838
<212> DNA
<213> Sus scrofa
<400> 44

```
tctggtcaat atgtagctca tctctaaaag gaacataggg ctccaatagg aggaccccag     60
tcttgtagtt aagtgtacct taactttttg cttcttcttt cttcttagga gctttaactt    120

aggaaatcta tcatcttgtt aaccctgaca aatgatttat cttcatcaat ctgtttaaac    180
ttgaagtcag aggctcaaat tattttctgt tttttcataa agttcagatt ttgagagact    240
ggttagcagc ttgtgtgcca atttaaggcc tttaaatgaa atactcaaaa ttctagattt    300
atcctaagtt taaaattgca aacctatact tcagctccac tctcccttca aatttttcta    360
cagaacctct gcaaagatag ggagactatc tgaccatacc aaagtataaa acattctaag    420
acaaccgaaa tggcagataa ttttcataaa grcccactaa tctctagtca tatatagagt    480
gaaatgaact tacaaaagtg aaaaatagat ccctagcaca ctgaccttaa aactgatcta    540
aatccataca tcaataggcc agacttggag ttcccatcat ggcacagtgg ttaaagaacc    600
cgactaggaa tcatcaggtt gcaggttcaa tccctggcct tgctcagtgg gttaagaatc    660
cagcattgct gtgagctgtg gtgtaggtcg cagacgtggc tcagattcca cgttgctgtg    720
gctctggcgt aggcgggagg ctacagctct gattagaccc ctcgcctaat atgccagggg    780
tgcagcccct cgcctaatat gccatgggtg cagccctaga aaagacaaaa aaaaaaaa      838
```

## Description

### FIELD OF THE INVENTION

The invention relates to methods for improving swine genetics and pork production and to compositions and kits useful to carry out such methods and to herds produced by said methods. Another aspect of the invention relates to the identification and use of a single nucleotide polymorphism in the porcine leptin receptor (LEPR) gene. The invention is also drawn to the use of probes to detect the LEPR gene polymorphism in order to identify those animals useful for as breeding stock for improved pork production.

### BACKGROUND OF THE INVENTION

The pork industry is experiencing phenomenal growth as it continues to meet worldwide consumer demand for what has become the meat product with the highest consumption. One key to maintaining industry growth and cost effective production is the continued implementation of high-quality standards into every level of the business.

In the United States, pork production is a vital part of the economy. Nearly 19 billion pounds were processed from about 97 million hogs in 2001. The economic impact of the industry on rural America is immense. Annual farm sales typically exceed $11 billion, while the retail value of pork sold to consumers reaches $38 billion each year.

Pork also provides employment well beyond the farm. The U.S. pork industry is responsible for over $72 billion in total domestic economic activity. In addition, the pork industry supports over 800,000 jobs and adds over $27 billion of value to basic production inputs such as corn and soybeans.

There are approximately 85,760 pork operations today compared to nearly three million in the 1950s. Farms have grown in size; nearly 80 percent of the hogs are grown on farms that produce 5,000 or more hogs per year.

Major technological advancements have allowed for production to grow dramatically over the years. A number of innovations, including the use of genetic capabilities for higher reproductive efficiencies and enhanced lean muscle growth, capturing economies of size, and developing animal management methods that have controlled diseases, have led to improved productive efficiency. In addition, U.S. pork producers are increasingly using state-of-the-art innovations designed to provide an environmentally efficient operation that ensures safe, high quality food for consumers.

There is also an increasing consumer demand for meat products with specific qualities, (e.g., low fat content). This demand is fueled by accumulating evidence in the scientific literature that a high consumption of animal fat, especially fat with a high proportion of saturated fatty acids, represents a significant health hazard, including risk for cardiovascular disease. Another health concerns associated with high fat meats is their high cholesterol content.

Faced with larger average farm size and consumers who seek a healthier meat product at a minimum cost, pork producers are continually pressed to reduce the cost of production and offer healthier products to stay competitive.

One tool used in pork production is the use of genetic differences that exist among individual meat producing animals as well as among pig breeds. These differences can be exploited by breeding techniques to achieve animals with these desirable characteristics. For example, Chinese breeds are known for reaching puberty at an early age and for their large litter size. In contrast, European and American breeds are known for their greater growth rates and leanness.

The occurrence of desirable traits (e.g. growth rate or muscle mass) in an animal and/or herd may be optimized by identifying those genes or genetic loci associated with variation in a particular trait of interest and increasing the incidence of the desirable allele of that gene or locus within a given pig population. This is necessary because the heritability for desired traits may be quite low. For example, heritability for litter size is around 10%-15%. Standard breeding methods that select individuals based upon phenotypic variations do not take into account genetic variability or complex gene interactions which may exist. Consequently, an improved approach that incorporates analysis of variation in an animal's DNA is desirable. Such a method provides a means for genetically evaluating animals to enable breeders to more accurately select those animals that not only phenotypically express desirable traits but also have the underlying favorable genetics. In theory, this can be accomplished by marker assisted selection.

RFLP analysis has been used by several groups to analyze pig DNA. Jung et al., Theor. Appl. Genet., 77: 271-274 (1989), discloses the use of RFLP (restriction fragment length polymorphisms) techniques to show genetic variability between two pig breeds. Polymorphisms were demonstrated for swine leukocyte antigen (SLA) Class I genes in these breeds. Hoganson et al., Abstract for Annual Meeting of Midwestern Section of the American Society of Animal Science, Mar. 26-28, 1990, incorporated herein by reference, reports on the polymorphism of swine major histocompatibility complex (MHC) genes for Chinese pigs; these were also demonstrated by RFLP analysis. Jung et al. Animal Genetics, 26:79-91 (1989), reports on RFLP analysis of SLA Class I genes in certain boars. The authors state that the results suggest that there may be an association between swine SLA/MHC Class I genes and certain production and performance traits. They further state that the use of SLA Class I restriction fragments, as genetic markers, may have potential in the future for improving pig growth performance.

In order to exploit the advantages of a specific favorable genetic allele one must first identify at least one genetic marker for each desired trait. The marker(s) may be linked to a single gene or to a number of genes, providing additive effects. DNA markers have several advantages; segregation is easy to measure and is unambiguous. Moreover, DNA markers are co-dominant, allowing all genotypic classes to distinctly identified. Also, DNA marker information can be assessed at an early age (prior to expression of the phenotype of interest) and markers for sex-linked and sex-influenced traits can be measured in both sexes.

The use of genetic differences in receptor genes has become a valuable marker system for selection. For example U.S. Pat. Nos. 5,550,024 and 5,374,526 to Rothschild *et. al.* disclose a polymorphism in the pig estrogen receptor gene that is associated with larger litter size, the disclosure of which is incorporated herein by reference. Another example is provided by U.S. Pat. No. 5,935,784, filed August 10, 1999, which discloses polymorphic markers in the pig prolactin receptor gene that are associated with larger litter size and overall reproductive efficiency.

The leptin receptor (LEPR) gene encodes the leptin receptor protein, which is a cytokine receptor that specifically recognizes the ligand "leptin." Upon binding its ligand the leptin receptor initiates a cellular signal transduction cascade that ultimately produces major physiological results, most significantly suppression of appetite. Expression variation in LEPR has been found in different nutritional states (Dyer *et al.).* Reviews of known functions of leptin and the leptin receptor are provided in Barb *et al.* and Tartaglia.

The porcine LEPR gene has been localized to chromosome 6, at approximately 122 centiMorgans (cM). Moreover, a number of DNA sequences (genomic and cDNA) for the porcine LEPR gene are available from the Genbank public DNA database, including: accession numbers: AF092422 (Ruiz-Cortez *et al.*), AF167719 (Hu *et al.),* AF184173, AF184172 and AH009271 (Lacroix *et al.),* AJ223163 and AJ223162 (Stratil *et al.*), U72070 (Ernst *et al.),* AF036908 (Matteri, R.L.), and U67739 (Matteri, R.L. and Carroll, J.A.).

The murine autosomal recessive mutations obese (*OB*), diabetes (*DB*) and fatty (*FA*) were first reported in the 1960s. The phenotypes of animals homozygous for these mutations include severe, early-onset obesity, insulin resistance and susceptibility to diabetes. The OB gene has recently been cloned in human and mouse and its protein product identified as leptin. Subsequent research led to the identification of a receptor for leptin in mice (OBR). The gene for OBR was shown to map to within a 5.1 cM interval of mouse chromosome 4 that also contains the *db* locus. This report was followed by two studies providing evidence that *db* is the gene encoding OBR. A recent report by Chua and associates has confirmed that *db, fa* and *obr* are all mutations of the same gene. The mouse leptin receptor gene has now been assigned the symbol, *Lepr,* which replaces the previously used symbols *OB-R* and *obr.* Mapping of human leptin receptor gene (LEPR) has also recently been reported.

The leptin receptor in mice (and humans) is a class-I transmembrane cytokine protein existing in two forms (*i.e.,* forms having either a short or a long cytoplasmic domain). Only the long form is believed to be capable of signal transduction. In mice, the LEPR gene product is believed to bind leptin (the 146 amino acid protein secreted into the blood by fats cells) in a 1:1 ratio (Devos *et al.,* Dyer *et al.,* Tartaglia). Administration of leptin to *ob*/*ob* mice, which are deficient in the production of leptin, causes a reduction in food intake and weight loss (Devos *et al.),* In ewes the LEPR is expressed in the anterior pituitary and adipose tissues. Moreover, it is differentially expressed in well-fed versus feed-restricted ewes (Dyer *et al.)*.

It has been hypothesized that various polymorphisms in pLEPR may affect commercially significant traits. For example, U.S. Pat. No. 6,458,531 (Rothschild et al.*),* Strait *et al.* and Vincent *et al.* describe genetic markers, based upon polymorphisms in and around the pLEPR gene. These polymorphisms are described as relating to leanness in pigs. The Rothschild *et al.* '531 patent suggests that use of the pLEPR markers described therein would permit genetic typing of pigs for their pLEPR allelic variants and for determination of the relationship of specific RFLPs to leanness. Thus, it is suggested that the described markers may be used as a selection tool in breeding programs to develop lines and breeds that produce litters containing offspring with less fat content.

However, none of the pLEPR polymorphisms described thus far are believed to cause any variance in the protein encoded by the pLEPR gene. Moreover, no determination of their nature (other than the fact that they are restriction fragment length polymorphisms) has been reported.

Study of the mouse LEPR indicates that the leptin binding domain resides in amino acid residues 323-640. Furthermore, co-expression of the active form of the receptor with an inactive mutant indicates that in its functional form the receptor may exist as a multimeric complex in the absence of leptin (Ming *et al.*).

Ovilo *et al.* have investigated the LEPR gene as possibly affecting carcass composition in pigs. When testing the RFLP previously published by Stratil *et al.* they confirmed an association between that polymorphism and fatness, but concluded that the RFLP was merely in some level of linkage disequilibrium with the causal mutation. The authors attempted to test the strength of association between carcass composition traits and the two RFLPs described in the Rothschild *et al.* '531 patent, but could not find an association because the polymorphisms did not occur frequently enough in the population tested.

In view of the discussion above it is likely that both leptin and the leptin receptor product play some part in the determination of body composition, fatness, muscle leanness, and feed intake in swine. Therefore, there exists a need to identify genetic markers that are linked to the expression of desirable commercial traits in pigs. There is also a need for methods of identifying the presence of absence of these markers in individual animals and of using these markers as part of a pig management or pig breeding program for the improvement of pork production. The invention described herein satisfies these needs.

## SUMMARY OF THE INVENTION

To meet the needs described above, the present invention provides a method of genotyping one or more non-human animals for selecting traits capable of modulating product quality and/or productivity comprising:

a) in a biological sample obtained from at least one non-human animal;
b) detecting at least one polymorphism in the porcine leptin receptor (pLEPR) gene; wherein the polymorphism causes a polymorphism in the pLEPR protein, wherein the polymorphism is a threonine/methionine polymorphism at amino acid number 69 of the prepro-pLEPR protein, and wherein the polymorphism results from the presence of either a thymidine (T) or cytidine (C) in the second position of the codon encoding amino acid number 69 of the prepro-pLEPR protein; and
c) establishing the genotype of the animal from which each biological sample was obtained; and
d) selecting the animal having the genotype to provide the selected trait,

wherein one or more of the traits are selected from the group consisting of average feed intake, average daily weight gain, muscle mass, back fat, water holding capacity, meat color, meat pH, intramuscular fat, meat tenderness, and/or cooking loss.

Various embodiments of the invention provide methods for detecting which allelic variant is present in a particular animal. These methods include, but are not limited to DNA sequencing, restriction fragment length polymorphism (RFLP) analysis, heteroduplex analysis, single strand conformational polymorphism (SSCP), denaturing gradient gel electrophoresis (DGGE), polymerase chain reaction (PCR), real time PCR analysis (TAQMAN®), temperature gradient gel electrophoresis (TGGE), primer extension, oligo-specific hybridization and INVADER® genetic analysis assays.

The INVADER® platform is based on a "perfect match" enzyme-substrate reaction. The INVADER® reaction uses proprietary CLEAVASE® enzymes, which recognize and cut only the specific structure formed during the Invader process. Instead of relying on target amplification, as in traditional methods, the INVADER® reaction generates its own signal amplification. In the INVADER® process, two short DNA probes hybridize to the target in the presence of the variation of interest to form the structure recognized by the CLEAVASE® enzyme. The enzyme then cuts one of the probes to release a short DNA flap. Each target can induce the release of several thousand flaps per hour. Each released flap can act as an Invader oligonucleotide on a FRET (fluorescence resonance energy transfer) cassette to create another structure recognized by the CLEAVASE® enzyme. If recognition occurs, the CLEAVASE® enzyme cuts the labeled probe, which emits a detectable fluorescent signal. Each flap generates thousands of signals per hour, yielding millions of detectable signals per target. The INVADER® reaction results are easily read on most existing fluorescence detection systems. If the variation in question is not present, then there is no overlap with the probe, the INVADER® oligo, and the target DNA. Hence, there is no recognition by the CLEAVASE® enzyme, and no flap is released. In the absence of the cleaved flaps, no invasive structure is formed, which means that no fluorescent signal is released from the FRET cassette. (INVADER® and CLEAVASE® are registered trademarks of Third Wave Technologies Inc., Madison, Wisconsin).

Disclosed herein also are kits comprising the components necessary to carry out methods for identifying polymorphisms in the LEPR gene. These kits comprise the components necessary to carry out any type of analysis suitable to detect the polymorphisms described herein. For example, analytical methods contemplated as being useful for the instant invention include, but are not limited to, the following: DNA sequencing, restriction fragment length polymorphism (RFLP) analysis, heteroduplex analysis, single strand conformational polymorphism (SSCP), denaturing gradient gel electrophoresis (DGGE), polymerase chain reaction (PCR), real time PCR analysis (TAQMAN®), temperature gradient gel electrophoresis (TGGE), primer extension, oligo-specific hybridization, and INVADER® assays. Any other suitable means for analyzing the structure of nucleic acids is also within the scope of the present invention.

Also contemplated as part of the instant invention are methods for detecting the allelic variation at the level of protein.

The necessary novel reagents for the kits mentioned above are also disclosed. Various aspects of this embodiment provide for oligonucleotide primers suitable for use as a DNA and/or RNA probes or as primers for DNA and/or RNA synthesis. Antibodies useful for detecting proteins produced from the allelic variant provided herein are also disclosed

One embodiment provides for a method for producing pigs and the pigs produced by that method (considered both as individuals and as a herd). Generally, the method comprises analyzing either one or a plurality of pigs and determining which form(s) of the pLEPR polymorphism each animal possesses. Next, this information is used as part of a breeding plan to produce one or more pigs having the desired qualitative and/or quantitative traits.

According to one aspect of this embodiment of the invention the method for producing pigs is employed to provide pigs having more desirable characteristics with respect to economic traits selected from, but not limited to, one or more of the following: the average feed intake and/or average daily weight gain, backfat, muscle mass, water holding capacity, meat color, intramuscular fat, meat tenderness, and/or cooking loss, the method comprising

a) screening a plurality of pigs to identify the nature of an allelic variant in the porcine leptin receptor (pLEPR) gene, wherein said allelic variant produces a threonine or methionine polymorphism at amino acid number 69 of the prepro-pLEPR protein;
b) selecting those pigs having a desired allele; and
c) using the selected pigs as sires/dams in a breeding plan to produce offspring; wherein the offspring have an increased frequency of the desired allele.

Another embodiment of the instant invention provides a method for increasing meat production in a herd by a method comprising modifying the herd genetics (*e.g.* the frequency of a particular LEPR gene allele) as provided herein as per claim 4. One aspect of this embodiment of the invention provides for a method wherein the EBV of the herd is improved over time with respect to the trait of meat production by, for example, increasing the frequency of the LEPR gene allele which has been shown to be linked to increased meat production.

**DESCRIPTION OF THE FIGURE**

The following figures forms part of the present specification and is included to further demonstrate certain aspects of the present invention. The invention may be better understood by reference to this figure in combination with the detailed description of specific embodiments presented herein.

| Figure | Description |
|---|---|
| 1 | DNA sequence (SEQ ID NO:10) and Amino Acid sequence (SEQ ID NO:11) of the portion of the pLEPR gene which contains the M69T and S73I polymorphisms. Primer sequences are underlined, the single nucleotide polymorphisms and accompanying amino acid changes are shown in bold. Nucleotide sequence without accompanying amino acid sequence is intronic. The forward primer starts at position 311 of Genbank accession AF184172, " Sus scrofa leptin receptor (LEPR) gene, exon 4 and partial cds". The M69T polymorphism is at nucleotide position 609 of AF184172. |

**DETAILED DESCRIPTION OF THE INVENTION**

The following definitions are provided in order to aid those skilled in the art in understanding the detailed description of the present invention.

As used herein the term "average feed intake" refers to the average amount of food consumed by an animal or animals during a defined "period". The definition may be used with reference to an individual animal or, alternatively, to a group of animals such as a litter, group of litters, or an entire herd. The "period" may include definite time periods such as intake per day, week, or month. Alternatively, it may be used, for example, to refer to average feed intake consumed by a group of animals by the time they reach a specified stage (e.g., "weanling", "grower", or "finisher").

As used herein the term "backfat" denotes a measurement of the thickness of the fat, as measured on the carcass (or by ultrasound prior to slaughter), at a specified point on the animal's back.

As used herein the term "BLUP" (which is an acronym for best linear unbiased prediction) refers to any of the various commercially available computer programs that are used for genetic evaluation of an animal and/or herd. Typical input parameters and data for BLUP programs include genetic parameter estimates, phenotypes and pedigrees.

As used herein the term "breeding plan" preferably refers to a program for improving herd genetics, including the average estimated breeding value (EBV) for the herd, using the methods provided herein. The "breeding plan" may employ the use of statistical models and/or computer programs, such as BLUP, to formulate the most effective means to achieve the desired genetic improvement and/or allelic frequency in the herd.

As used herein the term "cooking loss" preferably refers to the difference in weight, due to water loss, between a piece of meat before and after cooking..

As used herein the term "economic trait locus" (ETL) preferably refers to a location on a chromosome that is linked to "quantitative trait" providing economic value.

As used herein the terms "efficient growth traits" and/or "performance traits" preferably refers to a group of traits that are related to growth rate and/or body composition of the animal. Examples of such traits include but are not limited to average daily gain, average daily feed intake, feed efficiency, back fat thickness, loin muscle area, and lean percentage.

As used herein the term "estimated breeding value" (EBV) preferably refers to a specific numeric value for an animal that predicts its "breeding value". EBV is often calculated using commercially available analysis programs (the output from BLUP is an example of an EBV).

As used herein the term "fixing a genotype" preferably means producing a population of pigs that are all homozygous for the same allele of a particular marker in a specific gene or at a specific locus.

As used herein the term "gene" refers to a sequence of DNA responsible for encoding the instructions for making a specific protein within a cell (including when, where, and in what abundance the protein is expressed).

As used herein the term "intramuscular fat" refers to a measure of the fat content of a specific cut of meat (e.g. loin or ham) that is determined by chemical analysis.

As used herein the term "linkage disequilibrium" refers to: a non-random association of alleles at two or more loci. A quantitative measure of linkage disequilibrium is correlated to the probability of two alleles (at separate loci) being inherited together.

As used herein the term "locus" refers to a specific location on a chromosome (e.g. where a gene or marker is located). "Loci" is the plural of locus.

As used herein the term "locus group" preferably refers to any combination of two or more SNP (single nucleotide polymorphism) loci within approximately 5 cM of each other, irrespective of order.

As used herein the term "marker" refers to a sequence of DNA that has a specific location on a chromosome that can be measured in a laboratory. To be useful, a marker needs to have two or more alleles. Common types of markers include, but are not limited to: RFLP = restriction fragment length polymorphism; SSR = simple sequence repeat (a.k.a. "microsatellite" markers); and SNP = single nucleotide polymorphism. Markers may be either located within a known gene, or in apparently non-coding regions and not directly associated with a known gene.

As used herein the preferred meaning for the term "marker assisted allocation" (MAA) is the use of phenotypic and genotypic information to identify animals with superior estimated breeding values (EBVs) and the further allocation of those animals to a specific use designed to improve the genetic merit of breeding animals for sale or to improve the genetic value of the herd. "Allocation" refers to any form of animal management, the selection and distribution of breeding animals, including, but not limited to, marketing the animals as possessing desirable characteristics and shipping selected animals to other geographies. In a particular embodiment of the invention, "allocation" includes any decision, process, or action that is taken, initiated, or considered on the basis of the genetic merit of an animal or animals; where that genetic merit was influenced in any way by the genotypic information obtained from the LEPR locus of animal(s) or related animals. In this context, the phrase "related animals" refers to the process of genotyping an animals and then allocating offspring if/when the offspring's genotype could be predicted or assumed, and also covers the use of "allelic peeling" to estimate the genotype of ancestors based on the genotype of descendents.

As used herein the preferred meaning for the term "Marker assisted selection" (MAS) is the use of genotypic information in addition to more traditional phenotypic/pedigree information to identify animals with superior estimated breeding values (EBVs) for selection and use as breeding animals.

As used herein the term "meat color" is used to refer to a the color of uncooked loin and ham muscle scored either visually by a trained person using a color scale or objectively using a device to measure light reflectance from the cut surface of the meat. One visual industry standard is the Japanese Color Score which uses a six point system, with one being the lightest and six the darkest. Both Minolta L* and Hunter L* values are often measured objectively using instruments manufactured by Minolta Corp. and HunterLab, Inc., respectively. The L* values are measures of light reflectance from the cut surface of the meat. Higher L* values correspond to higher reflectance and lighter color.

As used herein the term "meat quality trait" preferably means any of a group of traits that are related to the eating quality (or palatability) of pork. Examples of such traits include, but are not limited to muscle pH, purge loss, muscle color, firmness and marbling scores, intramuscular fat percentage, and tenderness.

As used herein the term "meat tenderness" refers to quantitative evaluation of loin muscle tenderness as determined by the Warner-Bratzler shear force test. This measures the force required to shear a piece of meat of defined size and orientation that has been cooked to defined and controlled specifications.

As used herein the term "muscle mass" refers to the total amount of protein/muscle in the animal or carcass. Lean percentage is estimated and the numerator of the formula used is also an estimate of the total amount of fat-free lean in the carcass. Estimates of fat-free lean and lean percentage are functions of RTUS (real-time ultrasound) measurements of backfat (BF) and loin-eye area (LEA), both of which are typically measured in live animals, and body weight.

As used herein the term "polymorphism" refers to the variation that exists in the DNA sequence for a specific marker or gene. That is, by definition, in order for there to be more than one allele for a gene or marker a polymorphism must exist.

As used herein a "qualitative trait" is one that has a small number of discreet categories of phenotypes.

As used herein the term "quantitative trait" is used to denote a trait that is controlled by several genes each of small to moderate effect. The observations on quantitative traits are often assumed to follow a normal distribution.

As used herein the term "quantitative trait locus (QTL)" is used to describe a locus that contains polymorphism (s) that has an effect on expression of a quantitative trait.

As used herein the term "swine production herd" or "production herd" refers to a collection of animals whose primary purpose is to produce pigs that will be shipped to market for meat purposes.

As used herein the term "single nucleotide polymorphism (SNP) haplotype" preferably refers to a defined combination of SNP alleles from two or more SNP loci on one chromosome.

As used herein the term "water holding capacity" preferably refers to measurements of drip loss, purge, and cooking loss. The first two measurements are made on uncooked meat and measure the ability of the raw meat to hold water. The third measurement refers to cooking loss during preparation. It is generally desirable for pork to have low loss of water during storage and cooking.

According to various aspects of the instant invention the traits to be improved in the pig herd may be characterize as either "product quality traits" or "productivity traits". Product quality traits for which the instant invention is suitable for improvement include, but are not limited to: carcass measurements, meat water holding capacity, meat color, marbling, tenderness, and cooking performance. Productivity traits contemplated as part of the instant invention are typically made on live (growing) animals and are known to impact lean growth efficiency. Productivity traits contemplated as part of the instant invention include, but are not limited to: growth rate, backfat and loin muscle area, feed intake, muscle mass and feed efficiency.

One of ordinary skill in the art will understand that the product quality traits may be measured by a variety of methods. Methods that are known in the art include those that follow. Methods for making "carcass measurements" include, but are not limited to: hot carcass weight, carcass length, belly thickness, primal weights, Fat-O-Meter fat depth, and Fat-O-Meter loin depth. Methods for measuring "water holding capacity" include, but are not limited to: purge loss (7 day and 28 day), and drip loss (7 day and 28 day). Methods of evaluating "color" include, but are not limited to: determination of Hunter L* (loin), Minolta L* (loin), NPPC (National Pork Producers Council) loin color score, and Japanese color score (loin). In addition there is a strong correlation between loin and ham pH and measurements of water holding capacity and color score. Methods of evaluating "marbling", "tenderness", and cooking performance include, but are not limited to: NPPC loin marbling score, NPPC loin firmness score, percent intramuscular fat, percent moisture, cooking loss, and Warner-Bratzler shear force test.

As with "product quality traits", one of ordinary skill in the art will understand that "productivity traits" may be evaluated using a variety of techniques which are know in the art. Methods for evaluating growth rate include, but are not limited to: evaluating average daily gain over various intervals (*e.g.* birth to 196 days, 90 to 125 days, and 90 to 196 days). Backfat (BF) and loin muscle area (LEA) are often measured using real-time ultrasound (RTUS). The measurements are typically taken for at least two time points, so as to provide values for BF and LEA at specific days of production. Taking measurements at different times also allows for a calculation of the change in each of these values over the given interval. Feed intake is often measured over a determined interval. For example feed intake may be measured from day 90 to day 196. This allows cumulative feed intake over various intervals to be determined (e.g. days 90 to 196, days 90 to 104, days 90 to 118 and/or etc.) as well as average daily feed intake. Feed efficiency is nearly always estimated using measures of growth rate, BF, and LEA, and feed intake (if available).

To meet the need for genetic markers associated with desirable porcine traits, the present invention provides a method for screening pigs to determine those which will be likely to produce offspring with a desirable pork production traits. The method comprises: 1) obtaining a sample of genomic DNA from a pig; and 2) analyzing the genomic DNA obtained in 1) to determine which leptin receptor allele(s) is/are present and/or determine the animal's status with regards to alleles for any markers in linkage disequilibrium with informative markers in LEPR. The information collected by this method may then be used in preparing a breeding plan for increasing the frequency of the desired allele.

The instant invention is further drawn to methods comprising determining which variant of the pLEPR polymorphism is extant in an animal, or a plurality of animals, and then using this determination to formulate a breeding plan to increase the frequency of the desired allele and/or improve the quality of pig offspring produced.

It has been discovered that quantitative trait loci (QTLs) for backfat, muscle mass, average daily gain (ADG), water holding capacity, meat color, intramuscular fat (marbling), tenderness, and cooking loss have been discovered within the area of chromosome 6 where the LEPR gene is located (specifically between 100 cM and 130 cM). The inclusion of the pLEPR gene (at 122 cM) within the peak of these QTLs is consistent with an association with variation in this gene and the variation observed in the described traits *(see* Example 4).

Various embodiments of the current invention provides for the detection of variant alleles of one or more single nucleotide polymorphisms within the coding region of the LEPR gene that result in, or cause, a polymorphism in one or more amino acid residues of the protein product of the pLEPR gene. In preferred aspects of this embodiment of the invention the polymorphisms affect pig product quality traits and/or productivity traits. In even more preferred aspects of these embodiments the traits are selected from the group comprising, but not limited to feed intake, average daily gain, muscle mass, backfat, and water holding capacity, meat color, meat pH, intramuscular fat, meat tenderness, and cooking loss.

In one aspect of this embodiment of the present invention, the polymorphism comprises either a cytosine ("C") or thymine ("T") variant at the nucleotide corresponding to position 609 of Genbank accession AF184172 in the fourth exon of the pLEPR gene. This polymorphism produces a pLEPR protein having either a methionine (if the nucleotide is "T") or a threonine (if the nucleotide is "C") at amino acid number 69 of the prepro pLEPR protein. In the animals characterized thus far the "T" variant (containing thymine, encoding methionine) is most common *(see* Example 1). As a shorthand designator, the polymorphism will be referred to as "the T69M" polymorphism.

Various aspects of these embodiments of the invention provide methods for determining the genotype of a particular animal *(i.e.* genotyping the animal) with respect to the T69M polymorphism. That is, determining whether the animal is heterozygous or, alternatively, homozygous for one of the variants. These analytical methods include, but are not limited to DNA sequencing, primer extension, restriction fragment length polymorphism (RFLP) analysis, heteroduplex analysis, single strand conformational polymorphism (SSCP), denaturing gradient gel electrophoresis (DGGE), polymerase chain reaction (PCR) both simple and multiplexing (the simultaneous amplification of several sequences in a single reaction), real time PCR analysis (TAQMAN®), temperature gradient gel electrophoresis (TGGE), allele-specific hybridization, oligo-specific hybridization and INVADER® genotyping assays.

The current invention provides methods for the testing and/or selection of animals for a number of reasons including, but not limited to: breeding (animal husbandry), management, forensic purposes, and pedigree analysis.

In one embodiment the polymorphism may be identified by an RFLP assay. In one aspect of this embodiment the assay may comprise amplifying the pig leptin receptor gene from isolated pig genetic material; exposing the gene to a restriction enzyme that yields restriction fragments of the gene of varying length. The restriction fragments may then be separated by any suitable means. Contemplated methods for the separation of the restriction fragments so as to form a restriction pattern, include, but are not limited to such as by gel electrophoresis (e.g., using polyacrylamide or agarose gels) or HPLC separation. The resulting restriction fragment pattern from the animal is then compared with pig leptin receptor gene that is either known to have or not to have the desired marker. If a pig tests positive for the marker, such pig can be considered for inclusion in the breeding program. If the pig does not test positive for the marker genotype the pig can be culled from the group and used elsewhere.

In a preferred embodiment the gene to be analyzed is isolated and replicated using oligonucleotide primers and a DNA polymerase to amplify a specific region of the gene that contains the polymorphism. Next the amplified region is digested with a restriction endonuclease and the restriction fragments are separated. Visualization of the RFLP pattern is by simple staining of the fragments (for example with ethidium bromide), or by labeling either the primers or the nucleoside triphosphates used in amplification or both. In a particularly preferred aspect of this embodiment the DNA polymerase is a thermostable DNA polymerase such as *Taq, Pfu, Tfl,* or *Tli* DNA polymerase.

The kits for use in carrying out the methods for identifying polymorphisms in the pLERR gene comprise the components necessary to carry out any method of analysis suitable to detect the polymorphisms described herein or known to those of ordinary skill in the art.

At a minimum, the kit is a container with one or more reagents that identify a polymorphism either in or associated with the pLEPR gene (*e.g.* in linkage disequilibrium with the T69M locus). In one aspect of this embodiment of the invention the kit reagents may comprise a set of DNA and/or RNA oligonucleotide primers capable of amplifying a fragment of the pLEPR gene that contains the polymorphism. The kit further or alternatively comprise a restriction endonuclease enzyme that cleaves the pLEPR gene in at least one place. Other possible kit components include, but are not limited to, a DNA polymerase (which may be thermostable), a buffer, ribonucleotides and/or deoxyribonucleotides, a reverse transcriptase enzyme, and a fluorescent marker. Kits directed to detecting the protein product of the pLEPR gene might further comprise a radiomarker and/ one or more antibodies.

Other possible components are also considered herein. For example, kits comprising components necessary for an immunological assay to detect the allelic variant(s) of pLEPR at the protein level. Such components include, but are not limited to, the necessary antibodies, buffers, and/or labeling compounds required to perform an enzyme-linked immunosorbant assay (ELISA) or any other suitable immunoassay known to those of ordinary skill in the art.

The kit may comprise oligonucleotide primers suitable for use as a DNA and/or RNA probe or as a primer for DNA and/or RNA synthesis. Such oligonucleotides may comprise or consist of the sequences provided by one or more of the following: SEQ ID NO: 1, 2, and 4-9, or the complement of these sequences, or the RNA version of these sequences (wherein "U" is substituted for "T").

Other embodiments of the instant invention provide for methods for producing pigs. Generally, the methods comprises analyzing one or more pigs and determining which allele or alleles of one or more pLEPR polymorphism(s) each animal possesses. This information regarding the allelic composition of the analyzed animal is used as part of a method of managing a pig population.

In certain aspects of this embodiment of the invention the information collected from the analysis of the pLEPR gene in the analyzed pigs in tabulated and utilized, either in isolation or in conjunction with other genotypic and/or phenotypic information. In one particular aspect of the invention the tabulated information is used as part of a program of marker assisted selection, to identify animals with superior estimated breeding values for selection and use as breeding animals.

In another aspect of this embodiment of the invention the tabulated pLEPR information is used as part of a program of marker assisted allocation in order to improve the genetic merit of animals to be sold as breeding stock or to improve the genetics of the herd (for example to enhance the average estimated breeding value of the herd).

In certain aspects of these embodiments of the invention the method includes a breeding plan to produce one or more offspring having the desired allelic composition so as to provide the qualitative and/or quantitative traits sought. In various aspects of this embodiment the information can be used either with or without the assistance of a statistical model/program such as BLUP (best linear unbiased prediction) to determine the most effective means to obtain animals having the desired traits. In addition to algorithms like BLUP, any other means for determining which animals should be bred to each other and/or how the animals should be allocated for use in the breeding plan or in the herd, are contemplated by the instant invention. In one particularly preferred aspect of this embodiment of the invention the method is used to enhance the accuracy of the estimated breeding value (EBV) for the animals in the herd.

The pig herd obtained have more desirable characteristics with respect to economic traits selected from, but not limited to, one or more of the following: average feed intake and/or average daily weight gain, backfat, muscle mass, water holding capacity, meat color, intramuscular fat, meat tenderness, and/or cooking loss. The pigs are provided by any of the methods for producing pigs or managing pig populations described herein.

Various embodiments of the instant invention are drawn to altering the frequency of a pLEPR allele in a selected pig population. The method comprises screening a plurality of pigs to identify the nature of an allelic variant in the porcine leptin receptor (pLEPR) gene, wherein said allelic variant produces a threonine or methionine polymorphism at amino acid number 69 of the prepro-pLEPR protein. Such screening can be accomplished either by directly determining the DNA sequence or by any other suitable method, for example by determining the sequence of the pLEPR gene product protein or by identifying either a SNP or SNP haplotype known to be in linkage disequilibrium with a particular allelic variant. Once the nature of the polymorphism is known for each animal, then those pigs having the desired allelic makeup are selected. These pigs are then allocated for use according to a breeding plan designed to achieve the desired change in the pig populations allelic frequency. This breeding plan may be designed to increase the frequency of a particular allelic profile. One aspect of this embodiment of the invention includes employment of a plan to fix the allele in a given pig population. Alternatively, the breeding or managerial plan may be designed to decrease the allele or to provide for a more "balanced" occurrence of the allele.

Another embodiment of the instant invention provides a method for enhancing meat production (that is improving either the quality or the quantity of the meat) in a herd by a method comprising modifying the herd genetics (e.g. the frequency of a particular pLEPR gene allele) as provided herein. One aspect of this embodiment of the invention provides for a method wherein the EBV of the herd is improved over time with respect to the trait of meat production by, for example, increasing the frequency of the LEPR gene allele which has been shown to be linked to increased meat production.

The instant inventors have found an association between the T69M locus and various growth-related phenotypes (*see* Example 4). An analysis of 2625 pigs from a single commercial line, showed that the presence of the "C" allele had a statistically significant correlation with a positive effect on: early ADG (average daily gain from day 0 to day 90 of life); late ADG (average daily gain from day 90 to day 165 of life), loin muscle pH, and loin muscle color, and drip loss. There was a small negative effect of the "C" allele on backfat, *i.e.* backfat was slightly increased.

In addition, ninety seven (97) SNP markers, representing 38 loci on porcine chromosome 6 (SSC6) were genotyped on a panel of 1,442 pure line pigs from the same commercial line. The loci selected for SNP discovery were spread across an approximately 80 cM region on SSC6 which included the LEPR locus and the SNP producing the T69M mutation. Linkage disequilibrium analysis was used to identify both individual SNPs and SNP locus groups (for up to three adjacent/nonadjacent SNPs that locate within 1 centiMorgan (cM)) that were significantly associated with growth-related phenotypes (i.e. backfat thickness, leanness, off-test weight and weight gain). All 97 SNPs and possible locus combinations of two and three SNP located within 1 cM were assessed for association with all phenotypes. At least four SNPs (plus several locus groups containing these SNPs) were found to be significantly associated with backfat thickness, corrected for either age or weight. One of these SNPs included T69M and the other three mapped within 3 cM of T69M as estimated by linkage analysis (see Table 5).

One significant feature of the present invention is that it is drawn to a pLEPR gene polymorphism in the coding region of the gene and causes an amino acid change in the protein product of the pLEPR gene. In contrast, previously published polymorphisms are not
characterized as causing an amino acid change, instead they are believed to occur in the intronic, or non-coding, portion of the gene.

Other aspects of the instant invention provide for methods of identifying one or more single nucleotide polymorphism(s) in linkage disequilibrium with the T69M polymorphism as described in the EXAMPLES, below. Briefly, various aspects of this embodiment of the invention comprise identifying at least one large-insert genomic clone containing all or a portion of the pLEPR gene. This large-insert genomic clone may be obtained from a porcine genomic library and may be in any suitable format. Suitable formats include, but are not limited to, a bacterial artificial chromosome (BAC), a yeast artificial chromosome (YAC), P1, a cosmid, a fosmid, a phage, and a plasmid.

Next large-insert clones containing all or part of the pLEPR gene are identified by any suitable means. According to one aspect of this embodiment the clones are identified by hybridization with a DNA or RNA probe comprising all or a portion of the pLEPR gene. Once one or more clones comprising all or a portion of the pLEPR gene are identified then the sequence of all or a portion of such clones may be determined. In addition, sequences representing genes and expressed sequence tags (EST) and markers (e.g. microsatellites) that have been placed on either physical (e.g. radiation hybrid) or linkage maps and that appear to be in proximity to the pLEPR gene are identified and used to select BAC clones containing these sequences. Furthermore, sequences selected from comparative maps (e.g. human-porcine) that appear to be in the proximity of pLEPR can also be used to screen and select BAC clones. Following the sequencing of these clones, portions of the clones comprising regions in close proximity to the pLEPR gene can be identified, referred to herein as "target regions." In this context, "close proximity" refers to any chromosomal distance over which linkage disequilibrium may exist, preferably up to 5 cM (roughly equivalent to 5 million base pairs). Factors influencing linkage disequilibrium vary between populations and include effective population size, mating structure, generation interval, ancestry, and other factors. Once one or more target regions are identified a panel of animals is screened to determine the sequence of their genomes in the areas corresponding to the target regions. The data generated from this screening is then analyzed to identify any single nucleotide polymorphisms (SNPs) present therein. The nature of the T69M allelic variant is also determined for each of these animals. Finally, the SNP data is analyzed with respect to each newly identified SNP to determine which of the newly identified SNPs is in linkage disequilibrium with the T69M polymorphism.

According to various aspects of this embodiment of the invention, SNPs identified as being in linkage disequilibrium with the T69M polymorphism are useful as markers for use in any of the methods described herein.

## DESCRIPTION OF THE SEQUENCE LISTINGS

The following sequence listings form part of the present specification and are included to further demonstrate certain aspects of the present invention. The invention may be better understood by reference to one or more of these sequences in combination with the detailed description of specific embodiments presented herein.

| SEQ ID NO: | Description |
|---|---|
| 1 | LEPR-RFLP-F1 primer |
| 2 | LEPR-RFLP-R1 primer |
| 3 | Genbank accession no: AF184173 |
| 4 | LEPR-311-F |
| 5 | LEPR731-R |
| 6 | Forward primer for T69M TAQMAN® assay |
| 7 | Reverse primer for T69M TAQMAN® assay |
| 8 | Probe for T69M TAQMAN® assay |
| 9 | Probe for T69M TAQMAN® assay |
| 10 | Sequence from LEPR exon |
| 11 | LEPR sequence used to select BAC 335C21 |
| 12 | Sequence from BAC 335C21, which was used to identify informative SNPs |
| 13 | Sequence used to select BAC 036M15, which in close proximity to LEPR locus |
| 14 | Sequence from BAC 036M15, which was used to identify informative SNPs |
| 15 | Sequence used to select BAC 069P03 in close proximity to LEPR locus |
| 16 | Sequence from BAC 069P03, which was used to identify informative SNPs |
| 17 | Forward primer for TAQMAN® assay number 183482 |
| 18 | Reverse primer for TAQMAN® assay number 183482 |
| 19 | vicProbe for TAQMAN® assay number 183482 |

(continued)

| SEQ ID NO: | Description |
|---|---|
| 20 | famProbe for TAQMAN® assay number 183482 |
| 21 | Forward primer for TAQMAN® assay number 180851 |
| 22 | Reverse primer for TAQMAN® assay number 180851 |
| 23 | vicProbe for TAQMAN® assay number 180851 |
| 24 | famProbe for TAQMAN® assay number 180851 |
| 25 | Forward primer for TAQMAN® assay number 182553 |
| 26 | Reverse primer for TAQMAN® assay number 182553 |
| 27 | vicProbe for TAQMAN® assay number 182553 |
| 28 | famProbe for TAQMAN® assay number 182553 |
| 29 | Forward genomic primer derived from BAC clone 069P03 |
| 30 | Reverse genomic primer derived from BAC clone 069P03 |
| 31 | Forward genomic primer derived from BAC clone 036M15 |
| 32 | Reverse genomic primer derived from BAC clone 036M15 |
| 33 | Forward genomic primer derived from BAC clone 335C21 |
| 34 | Reverse genomic primer derived from BAC clone 335C21 |
| 35 | Forward primer to screen BAC library (705625F) |
| 36 | Reverse primer to screen BAC library(705625R) |
| 37 | Forward primer to screen BAC library (AR024A11F) |
| 38 | Reverse primer to screen BAC library(AR024A11R) |
| 39 | Forward primer to screen BAC library (3661588F) |
| 40 | Reverse primer to screen BAC library(3661588R) |
| 41 | Amplicon amplified using primers derived from BAC clone 069P03 |
| 42 | Amplicon amplified using primers derived from BAC clone 036M15 |
| 43 | Amplicon amplified using primers derived from BAC clone 335C21 |

The following examples are included to demonstrate preferred embodiments of the invention. It will be appreciated by those of skill in the art that the techniques disclosed in the examples which follow represent techniques determined by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

## EXAMPLES

### Example 1: Characterization of the "C"/"T" polymorphism in swine

PCR primers, corresponding to SEQ ID NO:4 and SEQ ID NO:5, were designed to amplify a portion of the LEPR gene containing a small intron and the beginning of the coding region. These primers were used as part of a PCR reaction using the DNA from 18 animals as template. The resultant amplicons were sequenced and analyzed for polymorphisms. This analysis resulted in the identification of a polymorphism at nucleotide 299 of the amplicon (corresponding to position 609 of Genbank accession AF184173, SEQ ID NO:3).

The set of 18 animals used for polymorphism discovery were from a first ("Line A"; Pietrain) and a second ("Line B"; Duroc) commercial line. DNA was extracted from either ear or tail tissue using commercially available DNA extraction materials (Qiagen N.V., Venlo, Netherlands). DNA was subjected to PCR amplification using oligonucleotide primers SEQ ID NO: 4 and SEQ ID NO: 5. Amplified fragments were sequenced in both directions using the amplification primers. Resultant DNA sequences were called, aligned, and characterized for polymorphism using the Phred/Phrap/Consed/Polyphred software package developed and distributed by Phil Green (University of Washington, Seattle, Washington). The polymorphism described herein was detected at position 299 of the amplified sequence, and was determined to alter the amino acid sequence of the LEPR protein.

**Example 2: Discovery of SNPs in close proximity to LEPR**

PCR primers were designed from a portion of the complete coding sequence of pLEPR (SEQ ID NO:11) from a porcine EST sequence that was in close proximity to pLEPR on a porcine radiation hybrid map (SEQ ID NO:13) and from a porcine EST sequence that was homologous to a sequence obtained from the human sequence map and in close proximity to human LEPR (SEQ ID NO:15). These PCR primers (SEQ ID NO:35-40) were used to screen BAC clones from a porcine bacterial artificial chromosome library (RPCI-44 see bacpac.chori.org/mporcine44.htm) and select a clone representing each sequence/locus (BAC clones 069P03, 036M15, and 335C21, respectively). These three BAC clones were then subcloned and approximately 48 subclones were randomly selected and sequenced. High quality subclone sequences that did not contain known porcine repetitive elements were then selected for another round of primer design. Due to the fact that only partial and often times non overlapping sequence was obtained for each of the selected BAC clones, the sequences selected for primer design usually did not include any of the original sequence (SEQ ID NO: 11, 13, and 15) used to screen the BAC library.

The genomic sequences derived from BAC clones 335C21, 036M15 and 069P03 and were used to identify SNPs in LD with the T69M polymorphism are represented by SEQ ID NOs: 12, 14, and 16, respectively. The PCR primers that were designed from these sequences and that were then used to amplify genomic DNA template from a panel of 18 animals are shown as SEQ ID NO:29-34. The sequences of the amplicons produced using these primers, together with the location of polymorphisms identified by aligning and comparing the sequences from each of the 18 animals used in the discovery panel are provided as SEQ ID NO:41-43.

The TAQMAN® SNP assay developed for the SNP identified in SEQ ID Nos:41, 42, and 43, were assigned assay numbers 183482, 180851, and 182553, respectively. The details for the primers and probes used these TAQMAN® assays are provided in tables 1-3.

**Table 1: Probes and primers for TAQMAN® assay number 183482**

| | |
|---|---|
| fwd Primer | 5'-GGCAGCTGTAACTGGTTACGAA-3' (SEQ ID NO:17) |
| rev Primer | 5'-TCGCAGCTCATATTGAATAACGATGT-3' (SEQ ID NO:18) |
| vicProbe | 5'-AAGTTCCAAATACTCTTTC-3' (SEQ ID NO:19) |
| vicAllele | G |
| famProbe | 5'-AAGTTCCAAATACTATTTC-3' (SEQ ID NO:20) |
| famAllele | T |

**Table 2: Probes and primers for TAQMAN® assay number 180851**

| | |
|---|---|
| fwd Primer | 5'-CAGACCCTCTGATATTTGGAAAAGCA-3' (SEQ ID NO:21) |
| rev Primer | 5'-GCCAGGATAATCATTTGAGTATAAGAAAAGAAC-3' (SEQ ID NO:22) |
| vicProbe | 5'-ACAGGAGCTACTAAAAT-3' (SEQ ID NO:23) |
| vicAllele | C |
| famProbe | 5'-CAGGAGCTATTAAAAT-3' (SEQ ID NO:24) |
| famAllele | T |

**Table 3: Probes and primers for TAQMAN® assay number 182533**

| | |
|---|---|
| fwd Primer | 5'-ACATTCTAAGACAACCGAAATGGCA-3' (SEQ ID NO:25) |
| rev Primer | 5'-CTAGGGATCTATTTTTCACTTTTGTAAGTTCATT-3' (SEQ ID NO:26) |

(continued)

| | |
|---|---|
| vicProbe | 5'-ATAATTTTCATAAAGACCCACTAAT-3' (SEQ ID NO:27) |
| vicAllele | A |
| famProbe | 5'-CATAAAGGCCCACTAAT-3' (SEQ ID NO:28) |
| famAllele | G |

**Example 3: Methods for Genotyping the T69M locus**

Several methods exist to determine allelic composition at the LEPR T69M polymorphism. Such methods include, but are not limited to, PCR amplification and sequencing using SEQ ID NO: 4 and SEQ ID NO: 5 or other suitable primer pairs consisting of DNA sequence flanking the polymorphism, RFLP analysis using amplification primers SEQ ID NO: 1 and SEQ ID NO: 2 or other suitable primers flanking the polymorphism in conjunction with a restriction endonuclease such as *BsrD*I or other suitable enzyme to discriminate between the "C" and "T" alleles in the amplified DNA, real time PCR analyses (TAQMAN ®) involving DNA amplification and probe hybridization where the hybridization probes are labeled and discriminate between the allelic forms, and other methods readily performed by those skilled in the art (see Table 4).

**Table 4**

| | |
|---|---|
| Fwd Primer | 5'-TTCAACTTTGAATGGACATGATGAG-3' (SEQ ID NO:6) |
| rev Primer | 5'-GTGGAAAGTTGTTTTAGAAGATAAGTTTGA-3' (SEQ ID NO:7) |
| vicProbe | 5'-TGTTGAAACGGAACTT-3'(SEQ ID NO: 8) |
| vicAllele | C |
| famProbe | 5'-TGTTGAAATGGAACTTA-3'(SEQ ID NO:9) |
| famAllele | T |

**Example 4: Association between the pLEPR (T69M) polymorphism with production- and meat quality-related traits**

To carry out the association analysis between the pLEPR (T69M) polymorphisms and production and meat quality traits, almost 3,000 "Line A" pigs, representing more than 100 paternal half-sib families, with a combination of production and meat quality trait records were genotyped.

Analysis was performed via the following steps. First, biologically impossible data and the phenotypes that were outside the range of mean plus/minus 4 phenotypic standard deviations were excluded. The phenotypes were then modeled to account for the effects of season-year-farm-building, sex, and age effect, and were excluded if their residuals were outside the range of residual mean plus/minus 4 residual standard deviations. Remaining phenotypes for all animals were pre-adjusted using the following two models:

$$\text{Phenotype} = \text{cgp} + \text{sex} + \text{age} + \text{sire} + \text{residual} \qquad [1]$$

$$\text{Phenotype} = \text{cgp} + \text{sex} + \text{age} + \text{sire} + \text{dam} + \text{residual} \qquad [2]$$

Where phenotype in model [1] denotes phenotype for daily gain of body weight for the whole growth period (WDA), daily gain of body weight for the test period (ADG2), backfat depth (BF) and loin eye area (LEA) measured at the $10^{th}$ rib on the day of off test, and phenotype in model [2] denotes daily gain of body weight for the period from birth to on test (ADG1); cgp represents contemporary group that was formed to account for season-year-farm-building effect; sire and dam are class variable to account for sire and dam effect. The association between the candidate gene polymorphism and pre-adjusted trait phenotypes vas evaluated using following model:

$$\text{Phenotype} = \text{genotype} + \text{residual}$$

**Phenotype = # allele + residual**

The effect of genotype or allele were estimated via a least squares procedure; and the p value was estimated based on an F distribution with the degrees of freedom equal to number of genotypes - 1 or number of alleles -1, respectively.

Not all animals had a complete set of phenotypic records, thus in Tables 5 and 6 the number of animals included in the analysis is indicated.

**Table 5. Deviation from adjusted population mean for the three genotypic classes of pLEPR T69M for production traits**

| | GENOTYPE | | | | |
|---|---|---|---|---|---|
| **Trait** | **C/C** | **C/T** | **T/T** | ***p*-value** | **# animals** |
| On test weight (lbs) | 7.09 | 3.51 | 1.72 | 0.0039 | 1056 |
| Off test backfat (inches) | 0.0109 | 0.0084 | -0.0046 | 0.0012 | 2590 |
| Off test lean percent | -0.272 | -0.149 | 0.059 | 0.0242 | 2589 |

**Table 6. Deviation from adjusted population mean for the three genotypic classes of pLEPR T69M for meat quality traits**

| | GENOTYPE | | | | |
|---|---|---|---|---|---|
| **Trait** | **C/C** | **C/T** | **T/T** | **p-value** | **# animals** |
| Loin Color (Hunter L* @ day 7) | -0.580 | -0.424 | 0.008 | 0.0158 | 388 |
| Loin pH @ day 7 | 0.032 | 0.011 | -0.013 | 0.0315 | 460 |

The predicted impact on average phenotypic values for weight gain, backfat, loin color, and loin pH by fixing the "C" allele in Line A boars was estimated *(see* Table 7). The initial frequency of the "C" allele in this boar line is 22% and changes in pure line offspring phenotypic values were estimated assuming frequency of the "C" allele were increased to 100%.

**Table 7. Impact of fixing "C" allele in terminal boar line on pure line offspring**

| **Trait** | **Predicted change** | **Percent of mean** | **P-value** |
|---|---|---|---|
| Gain on test<br>(lbs from 0-90 days)<br>(lbs from 90-165 days) | <br>+1.48<br>+2.01 | <br>1.4<br>1.4 | <br><0.05<br><0.001 |
| Backfat<br>(inches @ day 165) | <br>+0.01 | <br>2.3 | <br><0.001 |
| Loin color<br>(Hunter L* @ day 7) | <br>-0.375 | <br>1.0 | <br><0.05 |
| Loin pH @ day 7 | +0.021 | 0.36 | <0.01 |

Ninety seven (97) SNP markers, representing 38 loci on porcine chromosome 6 (SSC6) that were genotyped on a panel of 1,442 pure line pigs. Analysis was performed via the following steps: first, biologically impossible data and the phenotypes that were outside the range of mean plus/minus 4 phenotypic standard deviations were excluded. Next, phenotypes were modeled to account for the effects of season-year-farm-building, sex, and age effect, and were excluded if their residuals were outside the range of residual mean plus/minus 4 residual standard deviations. Remaining phenotypes for all animals of the entire pure line were pre-adjusted using the following two models:

$$\mathbf{Phenotype = cgp + sex + offage + residual}$$

$$\mathbf{Phenotype = cgp + sex + offwt + residual}$$

where cgp was designed to account for season-herd-building effect, offage and offwt denote age and body weight at off test (i.e., when measurements are taken), respectively.

The second step was to form locus groups, and to calculate probabilities for each possible haplotype pair for each animal. As a preparation, a linkage map for these 38 loci was constructed using genotype information of approximately 3000 animals, in combination with their radioactive hybridization information. Within each locus, the order of SNP was then arbitrarily assigned, and the linkage distance between adjacent within locus SNP was assumed to be 0.01 centiMorgan. Based on their linkage map information, every combination of 1 to 3 SNP markers that locate within the distance of 1 centiMorgan forms a locus group. For all 97 linked SNP, the probability of likely linkage phase of sires with SNP genotypes were calculated conditional on pedigree and the SNP genotypes of their parental, mate, and progeny genotype information, using a very efficient algorithm. Conditional on sire linkage phases, probability of each possible haplotype pair was calculated for each animal and for each locus group.

The third step was to evaluate the association between preadjusted trait phenotypes and haplotype pairs of animals using following model:

$$y_i = \sum_{k=1}^{K} \beta_k x_{ik} + e_i$$

where $y_i$ and $e_i$ are the preadjusted trait phenotype and the residue for animal i, respectively; $x_{ik}$ denote the sum of probability of both the paternal and maternal haplotype being k, $\beta_k$ is regression coefficient for haplotype k, and K is the total number of haplotypes in the population. The Type I error rate (p value) was estimated by performing 50,000 random permutations of phenotypes among paternal half-sibs. The results showed that at least four SNP markers (very tightly linked to the LEPR locus) and four locus groups that were significantly associated with backfat thickness. Table 8 shows the F-statistic, p value, linkage map position, frequency of the favorable allele and the estimated effect of fixing the favorable allele for these four SNPs. Table 9 shows the equivalent information for the four SNP haplotypes found to be significantly associated with backfat thickness. In the case of each of these SNP combinations, two haplotypes accounted for >99% of the observed genotypes, thus these haplotypes were essentially biallelic.

**Table 8. SNPs significantly associated with backfat thickness on SSC6**

| SNP/Assay # | Map position (cM) | F-statistic | p-value | Favorable allele frequency | Fixation effect |
|---|---|---|---|---|---|
| 180851 | 132.3 | 20.57 | <0.0001 | 0.719 | -0.00438 |
| 182553 | 133.3 | 21.18 | <0.0001 | 0.720 | -0.00443 |
| LEPR(T69M) | 133.3 | 20.67 | <0.0001 | 0.721 | -0.00436 |
| 183482 | 135.9 | 13.85 | 0.0001 | 0.486 | -0.0057 |

**Table 9. SNP haplotypes significantly associated with backfat thickness on SSC6**

| Locus Group ID No. | SNPs comprising haplotype | F-statistic | p-value | Favorable haplotype frequency | Fixation effect |
|---|---|---|---|---|---|
| 125 | 180851 + 182553 | 21.55 | <0.0001 | 0.870 | -0.00204 |

(continued)

| Locus Group ID No. | SNPs comprising haplotype | F-statistic | p-value | Favorable haplotype frequency | Fixation effect |
|---|---|---|---|---|---|
| 126 | 180851 + 182553 + LEPR(T69M) | 21.28 | <0.0001 | 0.872 | -0.00200 |
| 127 | 180851 + LEPR (T69M) | 21.34 | <0.0001 | 0.872 | -0.00200 |
| 129 | 182553 + LEPR (T69M) | 21.28 | <0.0001 | 0.873 | -0.00201 |

**Additional evidence to support the contention that LEPR polymorphisms are associated with productivity and meat quality traits.**

A resource population for QTL discovery was created by crossing Pietrain boars with Duroc sows. The F1 generation was intercrossed to produce a F2 generation in which alleles differing between the two founder lines were expected to be segregating. In total, 1,600 F2 progeny were generated and specific productivity and meat quality phenotypes were measured on at least 1,000 of these animals (depending on the specific trait). Approximately half of the F2 generation and their parents and grandparents were genotyped for 135 microsatellite markers spaced across all 18 autosomes. autosomes (9 microsatellite markers from SSC6).

Described below is a typical analysis procedure. First, performance trait phenotypes for all F2 animals were pre-adjusted using the following two models:

$$\text{Growth phenotype} = \text{cgp} + \text{sex} + \text{age} + \text{residual}$$

$$\text{RTUS phenotype} = \text{cgp} + \text{sex} + \text{offwt} + \text{residual}$$

where cgp was designed to account for season-herd-building-pen effect, and formation of cgp was different for different ages; age denotes the age when the measurement was taken; RTUS denotes real time ultrasound measurements of backfat or loin-eye area. For meat quality traits, phenotypes were pre-adjusted using following models:

$$\text{MQ1} = \text{sdate} + \text{residual}$$

$$\text{MQ2} = \text{sdate} + \text{sex} + \text{residual}$$

$$\text{MQ3} = \text{sdate} + \text{sex} + \text{sage} + \text{residual}$$

where MQ1 denotes drip loss after 7 days, drip loss after 28 days, or purge loss after 28 days; MQ2 denotes intramuscular fat, marbling score, or percent moisture; MQ3 denotes Warner-Bratzler shear force; sdate is slaughter date fitted as a class variable, and sage is slaughter age fitted as a covariate.

Second, grandparental line origin of F2 offspring was traced for each marker, as described by Haley et al. (1994). Two coefficients ($x_a$ and $x_d$) were calculated as the difference in probability of being homozygous and the probability of being heterozygous.

Third, the association between the preadjusted phenotype was evaluated as:

$$y_i = \beta_0 + \beta_a x_{ai} + \beta_d x_{di} + e_i$$

where $y_i$ is the preadjusted trait phenotype for animal i, $\beta_o$, $\beta_a$, and $\beta_d$ are regression coefficients. For each putative QTL position, residual sum squares is minimized to estimate regression coefficients and test statistic, and a chromosome was searched in incremental 1 cM steps, by performing analysis for each putative QTL position. Chromosomewise Type I error rate (p value) was estimated by performing 10,000 to 20,000+ random permutations of F2 phenotypes within each paternal half-sib family to determine empirically the proportion of times observed test statistics occurred by chance.

Porcine LEPR is closely linked (approximately 1 cM on MARC map) to SW1881, which was located at 121 cM on the linkage map constructed for the markers genotyped on SSC6. Tables 10 and 11 list traits that had a significant ($p < 0.05$) F-statistic at 122 cM for productivity and meat quality traits, respectively.

**Table 10. F-statistic and probability for productivity traits that had QTL coinciding with the predicted location of pLEPR**

| TRAIT | F-STATISTIC | ***p*-Value** |
|---|---|---|
| Average daily gain from d 0 to 56 | 13.21 | <0.0001 |
| Average daily gain from d 21 to 56 | 10.88 | <0.0001 |
| Average daily feed intake from d 90 to 196 | 12.31 | <0.0001 |
| Backfat at d 90 | 60.72 | <0.0001 |
| Backfat at d 124 | 73.40 | <0.0001 |
| Backfat at d 160 | 81.34 | <0.0001 |
| Backfat at d 196 | 75.90 | <0.0001 |
| Change in backfat from d 90 to 196 | 48.13 | <0.0001 |
| Change in loin eye area from d 125 to 196 | 7.73 | 0.0052 |

**Table 11. F-statistic and probability for meat quality traits that had QTL coinciding with the predicted location of pLEPR**

| TRAIT | F-STATISTIC | ***p*-Value** |
|---|---|---|
| Drip loss after 7 days | 6.79 | 0.0125 |
| Drip loss after 28 days | 5.59 | 0.0347 |
| Purge loss after 28 days | 6.77 | 0.0122 |
| Intramuscular fat | 31.45 | <0.0001 |
| Marbling score | 22.69 | <0.0001 |
| Percent moisture | 8.23 | 0.0047 |
| Warner-Bratzler shear force | 12.53 | <0.0001 |

Discovery of QTL for average daily gain, backfat and loin eye area in the F2 resource population closely associated with the predicted location of pLEPR on chromosome 6 corroborates the association between polymorphisms in pLEPR and growth rate, backfat and leanness discovered using "Line A" pigs. In addition, discovery of QTL for drip loss and purge loss in the F2 resource population closely associated with the predicted location of pLEPR on chromosome 6 also supports the association between pLEPR polymorphisms and loin color and pH. Although a QTL of large effect for loin pH and color score on chromosome 6 was not specifically identified by these particular analyses, these two traits are known to be highly correlated with drip loss and purge.

Genetic correlations between drip loss and pH, drip loss and color, and pH and color for data collected from our F2 animals were -0.66, 0.45, and -0.37, respectively. In addition, Huff-Lonergan *et al.,* 2002, reported significant (p <0.0001) phenotypic correlations between percent drip loss and pH and loin color score. These correlations are consistent with the observation that meat with a higher pH tends to be darker in color (lower L* values) and have less drip loss. Thus, in view of the information provided herein, it would be expected by one of skill in the art that polymorphisms in pLEPR would also be associated with drip loss and purge as well as loin color score and pH.

The association between pLEPR polymorphisms and intramuscular fat and meat tenderness did not exceed statistical significance, primarily because these phenotypes were not measured for the majority of animals from which DNA was taken for pLEPR T69M genotyping. However, backfat and percent intramuscular fat are highly positively correlated traits. Huff-Lonergan *et al.,* 2002, reported a significant association (0.45, p <0.0001) between $10^{th}$ rib backfat and percent lipid. In addition, Ovilo *et al.,* 2000 and de Koning *et al.,* 1999 also detected QTL for intramuscular fat on porcine chromosome 6. Therefore, based on the results provided herein, one of ordinary skill in the art would not find it surprising that the work disclosed herein also identifies QTL for intramuscular fat in the same location.

Genetic correlations between intramuscular fat and percent moisture and intramuscular fat and Warner-Bratzler shear force for data collected from our F2 animals were -0.83 and -0.63, respectively. These correlations are consistent with the observation that increased intramuscular fat decreases muscle protein and associated water and that meat tenderness increases (shear force decreases) with increased marbling or intramuscular fat. Thus, it is reasonable to assert that polymorphisms in pLEPR are associated with measurements of intramuscular fat, moisture and tenderness.

**Example 5: Identification of Single Nucleotide Polymorphism(s) (SNP(s)) in linkage disequilibrium with the pLEPR T69M SNP**

A person skilled in the art could discover Single Nucleotide Polymorphisms, (SNPs) in Linkage Disequilibrium (LD) with pLEPR T69M by processes similar to (but not limited to) the following:

The skilled artisan could identify a large-insert clone containing either the pLEPR gene or sequences in close proximity. Such a clone could be a bacterial artificial chromosome (BAC), yeast artificial chromosome (YAC), P1 phage, cosmid, fosmid, phage, or plasmid constructs. Obtaining this clone could involve hybridization to genetic libraries with labeled DNA or RNA probe, or by iterative PCR, using primers and/or probes known to amplify sequences at or near the pLEPR gene.

The molecule containing the pLEPR gene could then either be sequenced directly or be subcloned and then sequenced to identify specific DNA sequences known to exist in close proximity to (or flanking) the pLEPR gene. For the purposes of this example, these flanking sequences are referred to as "target" sequences. The number of target sequences obtained is relevant insofar as the presence of more target sequences proportionally increases the likelihood of identifying a SNP in LD with T69M.

Once target sequence is identified, primers suitable for use in Polymerase Chain Reaction (PCR) amplification of target DNA from a panel of animals (the "SNP discovery panel") could be designed. Target DNA derived from the SNP discovery panel could then be sequenced and any SNPs present in the discovery panel that are in LD with the T69M could be identified, if present. By definition, these SNPs would be physically located in close proximity to the pLEPR gene. The number of SNPs thus identified is relevant insofar as the discovery of more SNPs proportionally increases the likelihood of identifying a SNP in LD with T69M.

Once a set of SNPs to be tested for LD has been obtained, the skilled artisan could conduct experiments to calculate LD between T69M and the individual SNPs identified in the set. These experiments are conducted by identifying an independent panel of animals (the "LD panel") that are unrelated and representative of as many phylogenetically distinct breeds as practicable.

All animals within the LD panel could be genotyped for all SNPs within the set, as well as for the T69M SNP. Each genotype represents two alleles, one each from a distinct chromosome, one maternal and one paternal. Therefore, a "phase" relationship can exist between alleles at two loci. For example, assume two genes (A and B), each have two alleles (A1 and A2; B1 and B2), thus there are four combinations of alleles for each chromosome (A1-B1 A1-B2, A2-B1, and A2-B2). Most genotypes can be deconstructed to derive the two component chromosomes.

Linkage disequilibrium (LD) is then measured between two loci by using allele frequency data from the LD panel to calculate the expected frequency of the four combinations of alleles for each chromosome (A1-B1, A1-B2, A2-B1, and A2-B2) assuming random distribution (frequency of A1-B1 = frequency of A1 x frequency of B1, etc.). These frequencies are compared to the observed distribution of allele combinations. If the frequencies are significantly different (therefore non-random), the two loci are said to be in LD.

If two alternate combinations of alleles are never observed (for example, A1-B2 and A2-B1), then the two loci are said to be in complete LD. This situation provides the perfect opportunity to use one locus as a proxy for the second locus when genotyping animals.

**REFERENCES**

The following references provide exemplary procedural or other details supplementary to those set forth herein, Barb et al., Domestic Animal Endocrinology, 21:297-317 (2001).

de Koning, D.J., Janss, L.L.G., Rattink, A.P., van Oers, P.A.M., de Vries, B.J., Groenen, M.A.M., der Poel, J.J., de Groot, P.N., Brascamp, E.W. and van Arendonk, J.A.M. 1999. Detection of quantitative trait loci for backfat thickness and intramuscular fat content in pigs (Sus scrofa). Genetics 152: 1679-1690.

Devos et al., J. Biol. Chem., 272:18304-18310 (1997).

Dyer et al., Domestic Animal Endocrinology, 14:119-128 (1997).

Haley, C.S., S.A. Knott, and J.-M. Elsen, 1994. Mapping quantitative trait loci in crosses between outbred lines using least squares. Genetics 136: 1195-1207

Huff-Lonergan, E., Baas, T.J., Malek, M., Dekkers, J.C.M., Prusa, K. and Rothschild, M.F. 2002. Correlations among selected pork quality traits. Journal of Animal Science 80: 617-627.

Ming et al., Molecular Pharmacology, 53:234-240 (1998).

Ovilo et al., Genetic Sel Evo, 34:465-479 (Jul.-Aug. 2002).

Stratil et al., Animal Genetics, 29:405 (1998).

Tartaglia, J. Biol. Chem., 272:6093-6096 (1997).

Vincent et al., J. Anim. Sci., 75:2287 (1997)

## Claims

1. A method of genotyping one or more non-human animals for selecting traits capable of modulating product quality and/or productivity comprising:

    a) in a biological sample obtained from at least one non-human animal;
    b) detecting at least one polymorphism in the porcine leptin receptor (pLEPR) gene; wherein the polymorphism causes a polymorphism in the pLEPR protein, wherein the polymorphism is a threonine/methionine polymorphism at amino acid number 69 of the prepro-pLEPR protein, and wherein the polymorphism results from the presence of either a thymidine (T) or cytidine (C) in the second position of the codon encoding amino acid number 69 of the prepro-pLEPR protein; and
    c) establishing the genotype of the animal from which each biological sample was obtained; and
    d) selecting the animal having the genotype to provide the selected trait,

    wherein one or more of the traits are selected from the group consisting of average feed intake, average daily weight gain, muscle mass, back fat, water holding capacity, meat color, meat pH, intramuscular fat, meat tenderness, and/or cooking loss.

2. The method of claim 1 wherein the presence or absence of the polymorphism is determined by a method selected from the group consisting of: DNA sequencing, restriction fragment length polymorphism (RFLP) analysis, heteroduplex analysis, single strand conformational polymorphism (SSCP) analysis, denaturing gradient gel electrophoresis (DGGE), polymerase chain reaction (PCR) analysis, real time PCR analysis temperature gradient gel electrophoresis (TGGE), primer extension, allele-specific hybridization, INVADER® genetic analysis assays, and immunoassay; wherein the selected method uses one or more reagents selected from the group consisting of restriction endonuclease enzymes, DNA polymerases, reverse transcriptases, buffers and deoxyribonucleotides.

**3.** A method of enhancing a trait selected from the group consisting of: average feed intake and/or average daily weight gain, backfat, muscle mass, water holding capacity, meat color, meat pH, intramuscular fat, meat tenderness, and/or cooking loss of animals in a pig herd, the method comprising:

a) screening a plurality of pigs to identify the nature of an allelic variant in the porcine leptin receptor (pLEPR) gene, wherein said allelic variant produces a threonine or methionine polymorphism at amino acid number 69 of the prepro-pLEPR protein;
b) selecting those pigs having a desired allele; and
c) using the selected pigs as sires/dams in a breeding plan to produce offspring; wherein the offspring have an increased frequency of the desired allele.

**4.** A method of enhancing meat production from a swine herd comprising:

a) screening a plurality of pigs to identify the nature of an allelic variant in the porcine leptin receptor (pLEPR) gene, wherein said allelic variant produces a threonine or methionine polymorphism at amino acid number 69 of the prepro-pLEPR protein;
b) selecting those pigs having a desired allele;
c) using the selected pigs as sires/dams in a breeding plan to produce offspring, wherein the offspring have an increased frequency of the desired allele; and
d) repeating steps a) through c) until an increased allelic frequency for the desired allele is achieved.

**5.** A method of identifying a polymorphism associated with a trait selected from the group consisting of: average feed intake and/or average daily weight gain, backfat, muscle mass, water holding capacity, meat color, meat pH, intramuscular fat, meat tenderness, and/or cooking loss by identifying a single nucleotide polymorphism in linkage disequilibrium with the threonine/methionine polymorphism at amino acid 69 of the prepro-pLEPR protein (T69M polymorphism), the method comprising:

a) identifying at least one large-insert genomic clone containing all or a portion of the pLEPR gene;
b) determining the sequences of all or a portion of the clone(s);
c) identifying target regions in close proximity to the pLEPR gene;
d) screening a panel of animals to determine the sequence of the target regions;
e) identifying any single nucleotide polymorphisms (SNPs) present in the target regions to provide a set of at least one target SNP; and
f) determining which of the target SNPs is in linkage disequilibrium with the T69M polymorphism.

**6.** The method of claim 5 wherein the large-insert genomic clone is selected from the group consisting of a bacterial artificial chromosome (BAC), yeast artificial chromosome (YAC), P1 phage, cosmid, fosmid, phage, and plasmid constructs.

**7.** The method of claim 5 wherein the sequence of the clone is determined by a method comprising polymerase chain reaction amplification of a portion of the clone.

**8.** The method of claim 5 where the identified target regions are within 5 centiMorgans or 5 million base pairs of the pLEPR gene.

**9.** The method of claim 4 wherein part b) further comprises:

b') tabulating the identified nature of the allelic variance possessed by each pig; and
b") utilizing the tabulated variances as part of a program of marker assisted selection and/or marker assisted allocation.

**10.** The method of claim 1, wherein the presence or absence of the polymorphism is determined using an oligonucleotide suitable for use as a DNA or RNA probe or as a primer in DNA or RNA synthesis, wherein the oligonucleotide comprises a sequence selected from the group consisting of SEQ ID NO:1, 2, and 4-9.

**11.** The method of claim 10, wherein the oligonucleotide consists of a sequence selected from the group consisting of SEQ ID NO:1, 2, and 4-9.

12. The method of claim 2, wherein the selected DNA polymerase and/or reverse transcriptase are thermostable.

## Patentansprüche

1. Verfahren zur Genotyp-Erstellung von einem oder mehreren nicht-menschlichen Tieren, zum Auswählen von Merkmalen, die die Produktqualität und/oder die Produktivität modulieren, mit:

   a) einem biologischen Muster gewonnen von mindestens einem nicht-menschlichen Tier;
   b) der Erfassung von mindestens einem Polymorphismus in dem Schweine Leptin Rezeptor-Gen (pLEPR); wobei die Polymorphie einen Polymorphismus in dem pLEPR Protein verursacht, wobei der Polymorphismus ein Threonin/Methionin Polymorphismus bei der Aminosäure Nummer 69 des Prepro-pLEPR Proteins ist, und wobei der Polymorphismus sich aus der Anwesenheit von entweder einem Thymidin (T) oder Cytidin (C) in der zweiten Position der Kodon Codierung Aminosäure Nummer 69 des Prepro-pLEPR Proteins ergibt; und
   c) der Festlegung des Genotypen des Tieres, von dem jedes biologische Muster entnommen wurde; und
   d) der Auswahl des Tieres, das den Genotyp für die Bereitstellung des ausgewählten Merkmals besitzt,

   wobei ein oder mehrere der Merkmale aus der Gruppe bestehend aus durchschnittlicher Futtereinnahme, durchschnittlicher tägliche Gewichtszunahme, Muskelmasse, Rückenfett, wasserhaltender Kapazität, Fleischfarbe, Fleisch pH, intramuskuläres Fett, Fleischzartheit und/oder Verlust beim Kochen gewählt sind.

2. Verfahren nach Anspruch 1, wobei die Anwesenheit oder Abwesenheit von dem Polymorphismus durch ein Verfahren bestimmt wird, das aus der Gruppe bestehend aus DNA Sequenzierung, Restriktionsfragmentlängen-Polymorphismus (RFLP) Analyse, Heteroduplex Analyse, Einstrang Konformations- Polymorphismus (SSCP) Analyse, Denaturierungs-Gradient Gel-Elektrophorese (DGGE), Polymerase Kettenreaktion (PCR) Analyse, Realzeit PCR Analyse, Temperatur-Gradient Gel-Elektrophorese (TGGE), Primer Erweiterung, Allel-spezifische Hybridisierung, INVADER® genetische Analyse Assay, und Immunoassay gewählt wird; wobei das ausgewählte Verfahren ein oder mehrere Reagenzien gewählt aus der Gruppe bestehend aus Restriktionsendonuklease Enzym, DNA Polymerase, Revers - Transkriptasen, Puffern und Desoxyribonucleotiden verwendet.

3. Verfahren zur Verstärkung eines Merkmals ausgewählt aus der Gruppe bestehend aus durchschnittlicher Futtereinnahme, durchschnittlicher täglicher Gewichtszunahme, Muskelmasse, Rückenfett, wasserhaltender Kapazität, Fleischfarbe, Fleisch pH, intramuskuläres Fett, Fleischzartheit und/oder Verlust beim Kochen von Tieren in einer Schweinezucht, wobei das Verfahren Folgendes umfasst:

   a) Screening von einer Mehrzahl von Schweinen zur Identifizierung der Beschaffenheit einer Allel - Variante in dem Schweine- Leptin Rezeptor (pLEPR) Gen, wobei die besagte Allel - Variante einen Threonin- oder Methionin-Polymorphismus bei Aminosäure Nummer 69 des Prepro-pLEPR Proteins erzeugt;
   b) Auswahl der Schweine, die das erwünschte Allel besitzen; und
   c) Verwendung der ausgewählten Schweine als Vater- bzw. Muttertiere in einem Zuchtplan zur Erzeugung von Nachwuchs; wobei der Nachwuchs häufigeres Auftreten des erwünschten Alleles aufweist.

4. Verfahren zur Steigerung der Fleischproduktion von einer Schweineherde mit:

   a) der Screening von einer Mehrzahl von Schweinen zur Identifizierung der Beschaffenheit einer Allel - Variante in dem Schweine - Leptin Rezeptor (pLEPR) Gen, wobei die besagte Allel - Variante einen Threonin- oder Methionin-Polymorphismus bei Aminosäure Nummer 69 des Prepro-pLEPR Proteins erzeugt;
   b) der Auswahl der Schweine, die das erwünschte Allel besitzen; und
   c) der Verwendung der ausgewählten Schweine als Vater- bzw. Muttertiere in einem Zuchtplan zur Erzeugung von Nachwuchs; wobei der Nachwuchs häufigeres Auftreten des erwünschten Alleles aufweist.
   d) der Wiederholung der Schritte a) bis c) bis eine erhöhte Häufigkeit des erwünschten Alleles erreicht ist.

5. Verfahren zur Identifizierung eines Polymorphismus, das mit einem aus der folgenden Gruppe ausgewählten Merkmal verbunden ist: durchschnittlicher Futtereinnahme und/oder durchschnittlicher täglicher Gewichtszunahme, Muskelmasse, Rückenfett, wasserhaltender Kapazität, Fleischfarbe, Fleisch pH, intramuskuläres Fett, Fleischzartheit und/oder Verlust beim Kochen, durch Identifizierung eines Einzel- Nucleotiden Polymorphismus bei Aminosäure 69 des Prepro-pLEPR Proteins (T69M Polymorphismus), wobei das Verfahren Folgendes umfasst:

a) Identifizierung von mindestens einem breiten Insert genomischen Klon, welcher den ganzen oder einen Teil des pLEPR Gens enthält;
b) Bestimmung der Sequenzen des ganzen oder eines Teils des Klons bzw. der Klone;
c) Identifizierung von Zielbereichen in enger Nähe zu dem pLEPR Gen;
d) Screening einer Auswahl von Tieren zur Bestimmung der Sequenz der Ziehbereiche;
e) Identifizierung der vorhandenen Einzel Nucleotid-Polymorphismen (SNPs) in den Zielbereichen, zur Bereitstellung von einem Satz von mindestens einem Ziel SNP; und
f) Bestimmung, welches der Ziel SNPs in Verkettungs-Ungleichgewicht mit dem T69M Polymorphismus liegt.

**6.** Verfahren nach Anspruch 5, wobei der breite Insert genomische Klon aus der Gruppe bestehend aus künstlichem Bakterien - Chromosom (BAC), künstlichen Hefe-Chromosom (YAC), P1 Phage-, Kosmid-, Fosmid-, Phage-, und Plasmid-Konstrukten gewählt wird.

**7.** Verfahren nach Anspruch 5, wobei die Sequenz des Klons durch ein Verfahren bestimmt wird, welches Polymerase Kettenreaktion Verstärkung eines Teils des Klons umfasst.

**8.** Verfahren nach Anspruch 5, in dem der identifizierte Zielbereich innerhalb von 5 Centimorgans oder 5 Millionen Grundpaaren des pLEPR Gens liegt.

**9.** Verfahren nach Anspruch 4 wobei Schritt b) ferner Folgendes umfasst:

b') Tabellieren der identifizierten Beschaffenheit der Allele Varianz, welche jedes Schwein besitzt; und
b") Verwendung der tabellierten Varianzen als Teil eines Programms zur Markierung-unterstützten Auswahl und/oder Markierung-unterstützten Zuweisung.

**10.** Verfahren nach Anspruch 1, wobei die Anwesenheit oder Abwesenheit des Polymorphismus durch die Verwendung eines Oligonucleotid bestimmt ist, welches zur Verwendung als DNA oder RNA Probe oder als Premier in einer DNA oder RNA Synthese geeignet ist, wobei das Oligonucleotid eine Sequenz umfasst, welche aus der Gruppe bestehend aus SEQ ID Nr.:1, 2 und 4-9 gewählt wird.

**11.** Verfahren nach Anspruch 10, wobei das Oligonucleotid aus einer Sequenz besteht, welche aus der Gruppe bestehend aus SEQ ID Nr.:1, 2 und 4-9 gewählt wird.

**12.** Verfahren nach Anspruch 2, wobei die gewählte DNA Polymerase und/oder Revers - Transkriptase thermostabil sind.

## Revendications

**1.** Procédé de génotypage d'un ou plusieurs animaux non humains pour sélectionner des traits capables de moduler la qualité du produit et/ou la productivité, comprenant les étapes consistant à :

a) dans un échantillon biologique obtenu sur au moins un animal non humain ;
b) détecter au moins un polymorphisme dans le gène du récepteur de la leptine porcin (pLEPR) ; le polymorphisme provoquant un polymorphisme dans la protéine pLEPR, le polymorphisme étant un polymorphisme thréonine/méthionine au niveau de l'acide aminé numéro 69 de la protéine pré-pro-pLEPR, et le polymorphisme résultant de la présence d'une thymidine (T) ou d'une cytidine (C) en seconde position du codon codant l'acide aminé numéro 69 de la pré-pro-protéine pLEPR ; et
c) établir le génotype de l'animal sur lequel chaque échantillon biologique a été obtenu ; et
d) sélectionner l'animal ayant le génotype pour fournir le trait sélectionné,

dans lequel un ou plusieurs des traits sont choisis dans le groupe consistant en la prise alimentaire moyenne, la prise de poids quotidienne moyenne, la masse musculaire, le lard gras, la capacité de rétention d'eau, la couleur de la viande, le pH de la viande, la graisse intramusculaire, la tendreté de la viande et/ou la perte à la cuisson.

**2.** Procédé selon la revendication 1, dans lequel la présence ou l'absence du polymorphisme est déterminée par un procédé choisi dans le groupe consistant en : le séquençage de l'ADN, l'analyse de polymorphisme de longueur de fragment de restriction (RFLP), l'analyse d'hétéroduplex, l'analyse de polymorphisme de conformation monocaténaire (SSCP), l'électrophorèse sur gel avec gradient de dénaturation (DGGE), l'analyse par réaction de polymérisation en chaîne (PCR), la PCR en temps réel, l'électrophorèse sur gel avec gradient de température (TGGE), l'extension d'amorce, l'hybridation spécifique d'allèle, des tests d'analyse génétique INVADER®, et l'immunodosage ; le procédé choisi utilisant un ou plusieurs réactifs choisis dans le groupe consistant en des enzymes endonucléases de restriction, des polymérases de l'ADN, des transcriptases inverses, des tampons et des désoxyribonucléotides.

**3.** Procédé d'amélioration d'un trait choisi dans le groupe consistant en : la prise alimentaire moyenne et/ou la prise de poids quotidienne moyenne, le lard gras, la masse musculaire, la capacité de rétention d'eau, la couleur de la viande, le pH de la viande, la graisse intramusculaire, la tendreté de la viande et/ou la perte à la cuisson d'animaux dans un troupeau de porcs, le procédé comprenant les étapes consistant à :

a) examiner une pluralité de porcs pour identifier la nature d'un variant allélique dans le gène du récepteur de la leptine porcin (pLEPR), ledit variant allélique produisant un polymorphisme de la thréonine ou de la méthionine au niveau de l'acide aminé numéro 69 de la protéine pré-pro-pLEPR ;
b) sélectionner les porcs ayant un allèle désiré ; et
c) utiliser les porcs sélectionnés comme géniteurs/génitrices dans un plan de reproduction pour produire une descendance, la descendance ayant une plus forte fréquence de l'allèle désiré.

**4.** Procédé d'amélioration de la production de viande d'un troupeau de porc comprenant les étapes consistant à :

a) examiner une pluralité de porcs pour identifier la nature d'un variant allélique dans le gène du récepteur de la leptine porcin (pLEPR), ledit variant allélique produisant un polymorphisme thréonine ou méthionine au niveau de l'acide aminé numéro 69 de la protéine pré-pro-pLEPR ;
b) sélectionner les porcs ayant un allèle désiré ;
c) utiliser les porcs sélectionnés comme géniteurs/génitrices dans un plan de reproduction pour produire une descendance, la descendance ayant une fréquence accrue de l'allèle désiré ; et
d) répéter les étapes a) à c) jusqu'à ce qu'une fréquence allélique accrue pour l'allèle désiré soit atteinte.

**5.** Procédé pour identifier un polymorphisme associé à un trait choisi dans le groupe consistant en : la prise alimentaire moyenne et/ou la prise de poids quotidienne moyenne, le lard gras, la masse musculaire, la capacité de rétention d'eau, la couleur de la viande, le pH de la viande, la graisse intramusculaire, la tendreté de la viande et/ou la perte à la cuisson en identifiant un polymorphisme de nucléotide unique en déséquilibre de liaison avec le polymorphisme thréonine/méthionine au niveau de l'acide aminé 69 de la protéine pré-pro-pLEPR (polymorphisme T69M), le procédé comprenant les étapes consistant à :

a) identifier au moins un clone génomique à grand insert contenant tout ou partie du gène de la pLEPR ;
b) déterminer les séquences de tout ou partie du ou des clones ;
c) identifier des régions cibles à proximité étroite du gène de la pLEPR ;
d) examiner un panel d'animaux pour déterminer la séquence des régions cibles ;
e) identifier un polymorphisme de nucléotide unique (SNP) quelconque présent dans les régions cibles pour fournir un ensemble d'au moins un SNP cible ; et
f) déterminer lequel des SNP cibles est en déséquilibre de liaison avec le polymorphisme T69M.

**6.** Procédé selon la revendication 5, dans lequel le clone génomique à grand insert est choisi dans le groupe consistant en un chromosome bactérien artificiel (BAC), un chromosome de levure artificiel (YAC), le phage P1, un cosmide, un fosmide, un phage et des constructions de plasmide.

**7.** Procédé selon la revendication 5, dans lequel la séquence du clone est déterminée par un procédé comprenant l'amplification par réaction de polymérisation en chaîne d'une partie du clone.

**8.** Procédé selon la revendication 5, dans lequel les régions cibles identifiées se trouvent à une distance de 5 centimorgans ou 5 millions de paires de bases du gène de la pLEPR.

**9.** Procédé selon la revendication 4, dans lequel la partie b) comprend en outre :

b') la tabulation de la nature identifiée de la variance allélique possédée par chaque porc ; et
b") l'utilisation des variances tabulées dans le cadre d'un programme de sélection assistée par marqueur et/ou d'allocation assistée par marqueur.

**10.** Procédé selon la revendication 1, dans lequel la présence ou l'absence du polymorphisme est déterminée en utilisant un oligonucléotide adapté pour être utilisé comme une sonde d'ADN ou d'ARN ou comme une amorce dans la synthèse d'ADN ou d'ARN, l'oligonucléotide comprenant une séquence choisie dans le groupe consistant en SEQ ID N° 1, 2, et 4-9.

**11.** Procédé selon la revendication 10, dans lequel l'oligonucléotide consiste en une séquence choisie dans le groupe consistant en SEQ ID N° 1, 2, et 4-9.

**12.** Procédé selon la revendication 2, dans lequel la polymérase de l'ADN et/ou la transcriptase inverse choisies sont thermostables.

```
GCACTGTTTGAGCACTTGGAAAGTTAAATAATTATTGTTGGAGACTGCATGTTTTAATCTTAGA
TACTTCCTATTTATGTCTTAGTCAAAATGATTAATTGCTTTTCTATGTGTCTTTTAAATGTCCT
AACA GAA TTT ATT TAT GTG ATA ACT GCA TTT GAC TTG
                E   F   I   Y   V   I   T   A   F   D   L

GCA TAT CCA ATT ACT CCT TGG AAA TTT AAG TTG TCT TGC ATG CCA
 A   Y   P   I   T   P   W   K   F   K   L   S   C   M   P

CCA AAT ACA ACA TAT GAC TTC CTC TTG CCT GCT GGA ATC TCA AAG
 P   N   T   T   Y   D   F   L   L   P   A   G   I   S   K

AAC ACT TCA ACT TTG AAT GGA CAT GAT GAG GCA GTT GTT GAA
 N   T   S   T   L   N   G   H   D   E   A   V   V   E

A[T/C]G GAA CTT AAT [T/A][C/T]A  AGT GGT ACC TAC TTA TCA AAC
  M/T    E   L   N      S/I       S   G   T   Y   L   S   N

TTA TCT TCT AAA ACA ACT TTC CAC TGT TGC TTT TGG AGT GAG GAA
 L   S   S   K   T   T   F   H   C   C   F   W   S   E   E

GAT AAA AAC TGC TCT GTA CAT GCA GAC AAC ATT GCA GGG AAG G
 D   K   N   C   S   V   H   A   D   N   I   A   G   K
```

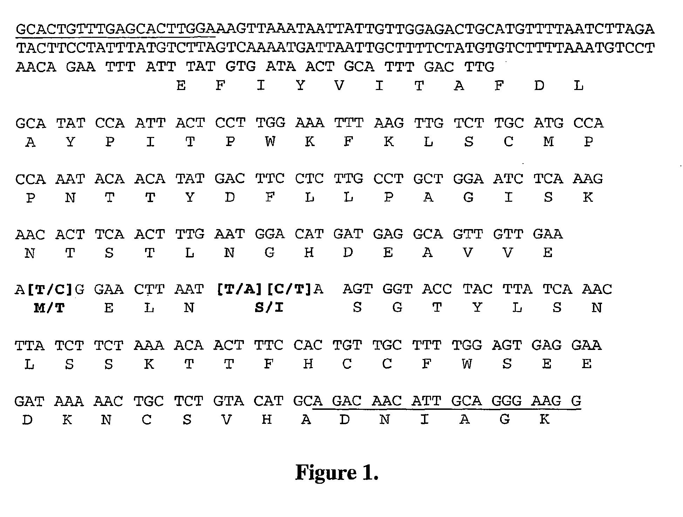

**Figure 1.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5550024 A
- US 5374526 A, Rothschild
- US 5935784 A
- US 6458531 B, Rothschild
- US 60553582 B
- US 60493158 B

### Non-patent literature cited in the description


- **JUNG et al.** *Theor. Appl. Genet.,* 1989, vol. 77, 271-274
- **HOGANSON et al.** *Abstract for Annual Meeting of Midwestern Section of the American Society of Animal Science,* 26 March 1990
- **JUNG et al.** *Animal Genetics,* 1989, vol. 26, 79-91
- **BARB et al.** *Domestic Animal Endocrinology,* 2001, vol. 21, 297-317
- **DE KONING, D.J. ; JANSS, L.L.G. ; RATTINK, A.P. ; VAN OERS, P.A.M. ; DE VRIES, B.J. ; GROENEN, M.A.M. ; DER POEL, J.J. ; DE GROOT, P.N. ; BRASCAMP, E.W. ; VAN ARENDONK, J.A.M.** Detection of quantitative trait loci for backfat thickness and intramuscular fat content in pigs (Sus scrofa). *Genetics,* 1999, vol. 152, 1679-1690
- **DEVOS et al.** *J. Biol. Chem.,* 1997, vol. 272, 18304-18310
- **DYER et al.** *Domestic Animal Endocrinology,* 1997, vol. 14, 119-128
- **HALEY, C.S. ; S.A. KNOTT ; J.-M. ELSEN.** Mapping quantitative trait loci in crosses between outbred lines using least squares. *Genetics,* 1994, vol. 136, 1195-1207
- **HUFF-LONERGAN, E. ; BAAS, T.J. ; MALEK, M. ; DEKKERS, J.C.M. ; PRUSA, K. ; ROTHSCHILD, M.F.** Correlations among selected pork quality traits. *Journal of Animal Science,* 2002, vol. 80, 617-627
- **MING et al.** *Molecular Pharmacology,* 1998, vol. 53, 234-240
- **OVILO et al.** *Genetic Sel Evo,* July 2002, vol. 34, 465-479
- **STRATIL et al.** *Animal Genetics,* 1998, vol. 29, 405
- **TARTAGLIA.** *J. Biol. Chem.,* 1997, vol. 272, 6093-6096
- **VINCENT et al.** *J. Anim. Sci.,* 1997, vol. 75, 2287